# EUROPEAN PATENT APPLICATION

(11) **EP 4 763 060 A2**
(43) Date of publication of application: **24.06.2026**
(21) Application number: 26176572.1
(22) Date of filing: 02.06.2022
(51) Int. Cl.: A61B 5/00

(54) **TRANSCUTANEOUS SENSOR APPLICATOR AND APPLICATOR ASSEMBLY**

(30) Priority: 05.08.2021 KR 20210103244
(62) Divisional of application: 22853253.7
(71) Applicant: i-Sens, Inc., Seoul 06646 (KR)
(72) Inventor: CHOI, Hyun Ho, 06646 Seoul (KR); RYU, Goang Yel, 06646 Seoul (KR); WANG, Ji Hoon, 06646 Seoul (KR)
(74) Representative: BCKIP Part mbB

(57) **Abstract**

An applicator according to the present disclosure is an applicator for inserting a sensor for measuring biometric information into a user's skin, the applicator comprising: an applicator body; an insertion unit installed on the applicator body to move a sensor unit from a first position in which same is spaced apart from the user's skin to a second position in which the sensor is inserted into the user's skin, the sensor unit comprising the sensor, a sensor unit housing on which the sensor is mounted, an adhesive layer provided on the sensor unit housing, and a protective sheet covering the adhesive layer; and a moving tab to which a part of the protective sheet is coupled, the moving tab being movably installed on the applicator body to be movable by the user such that the protective sheet can be moved away from the sensor unit housing and thus separated from the adhesive layer.

## Description

### TECHNICAL FIELD

The present disclosure relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of a user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

### SUMMARY

### Technical Problem

The present disclosure is developed in consideration of the above-mentioned points, and the purpose of the present disclosure is to provide an applicator and an applicator assembly for the transcutaneous sensor that minimize the additional work for a user to insert a transcutaneous sensor into a skin by enabling a sensor unit including the transcutaneous sensor to be manufactured in an assembled state, and can be conveniently used by a user when inserting the transcutaneous sensor into a skin.

Another purpose of the present disclosure is to provide an applicator and applicator assembly for a transcutaneous sensor that can easily remove the protective sheet that covers and protects the adhesive layer provided in the sensor unit or the adhesive layer provided in the base unit coupled with the sensor unit.

### Solution to Problem

To accomplish the purposes described above, according to an embodiment of the present disclosure, an applicator for inserting a sensor for measuring biometric information into skin of a user may comprise: an applicator body; an insertion unit installed to the applicator body to move a sensor unit, which includes the sensor, a sensor unit housing to which the sensor is mounted, an adhesive layer provided at the sensor unit housing, and a protective sheet covering an adhesive layer, from a first position spaced apart from the skin of the user to a second position where the sensor is inserted into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.

The moving tab may be installed to the applicator body to be movable in a direction of intersecting a movable direction of the sensor unit.

The moving tab may be installed to the applicator body to move in a direction perpendicular to a movable direction of the sensor unit.

An opening may be provided at one side of the applicator body, and the moving tab may be configured to move in a direction of intersecting a movable direction of the sensor unit such that at least a portion of the moving tab is drawn out from an inside of the applicator body to an outside of the applicator body through the opening.

The moving tab may comprise: a moving tab body located inside the applicator body and coupled with the protective sheet, and a handle portion extending from one side of the moving tab body and exposed to the outside of the applicator body for the user to be able to hold the handle portion with a hand.

A moving tab arm configured to be engageable with an applicator body detent provided at the applicator body may be provided to be elastically deformable at one side of the moving tab body, and the moving tab arm may be configured to, when a force greater than a preset amount is applied to the moving tab, be disengaged from the applicator body detent to be withdrawn from the opening.

The moving tab may be coupled to the protective sheet by an adhesive member.

The protective sheet may comprise: a protective portion covering the adhesive layer, a wing portion extended from an edge of the protective portion, and a protective sheet hole formed at one side of the wing portion, and the moving tab may have a grip portion corresponding to the protective sheet hole, and is coupled with the protective sheet in a way that the grip portion is inserted into the protective sheet hole.

The applicator according to an embodiment of the present disclosure may comprise: an operation member installed to the applicator body to be operated by the user; and a stopper member movably installed to the applicator body to operate the insertion unit in association with the operation member, and wherein the moving tab is configured to restrain movement of the stopper member in an initial position where the protective sheet is coupled with the adhesive layer to cover the adhesive layer, and release restraint on the stopper member in a removal position where the protective sheet is separated from the adhesive layer.

The applicator according to an embodiment of the present disclosure may comprise a locking unit including a locking body installed to the applicator body to be engaged with the stopper member or disengaged from the stopper member, the moving tab may be configured to bias the locking body to be engaged with the stopper member by contacting the locking body in the initial position, and the locking body may be configured to, when the moving tab moves to the removal position, be disengaged from the stopper member.

The locking body may compose: a body portion connected to the applicator body, wherein an angle of the body portion is changeable, a hook portion connected to one side of the body portion to be engaged with the stopper member, and a leg portion connected to another side of the body portion to contact the moving tab, and the locking body may be configured to, when the moving tab is located in the initial position, be biased in a direction in which the hook portion is engaged with the stopper member by contact of the leg portion with the moving tab, and the leg portion is configured to, when the moving tab moves to the removal position, move away from the moving tab and be biased in a direction in which the hook portion is disengaged from the stopper member.

The applicator body may include a stage which movably supports the stopper member, and the body portion is arranged in a through hole formed at one side of the stage to penetrate the stage.

The locking unit may comprise an elastic member configured to apply elastic force to the locking body in a direction of being away from the stopper member, and the locking body may be configured to, when the moving tab is located in the initial position, contact the moving tab to be biased in a direction of being engaged with the stopper member, and, when the moving tab moves to the removal position, deviate from the moving tab to be biased in a direction of being released from the stopper member by the elastic force of the elastic member.

The insertion unit may include a needle which is movable from the first position to the second position to be inserted into the skin of the user together with the sensor, and the moving tab may include a cover portion arranged between the protective sheet and the needle to prevent the needle from contacting the protective sheet.

In addition, to accomplish the purposes described above, according to an embodiment of the present disclosure, an applicator for inserting a sensor into skin of a user may comprise: an applicator body to which a base unit including a base unit housing, and an adhesive portion provided at the base unit housing to be attached to the skin of the user is separably coupled; an insertion unit installed to the applicator body to move the sensor unit, which includes the sensor and a sensor unit housing to which the sensor is mounted, an adhesive layer provided at the sensor unit housing to be attachable to the base unit housing, and a protective sheet covering the adhesive layer from a first position which is spaced apart from the base unit to a second position where the sensor unit is coupled with the base unit to insert the sensor into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.

Additionally, to accomplish the purposes described above, according to an embodiment of the present disclosure, an applicator for inserting a sensor into skin of a user may comprise: an applicator body to which a base unit including a base unit housing, an adhesive layer provided at one side of the base unit housing, a protective sheet covering the adhesive layer, and an adhesive portion provided at another side of the base unit housing to be attached to the skin of the user is separably coupled; an insertion unit installed to the applicator body to move the sensor unit, which includes the sensor and a sensor unit housing which is configured to be attachable to the base unit housing by the adhesive layer and the sensor is mounted to, from a first position which is spaced apart from the base unit to a second position where the sensor unit is coupled with the base unit to insert the sensor into the skin of the user; and a moving tab to which a portion of the protective sheet is coupled and is movably installed to the applicator body to be movable by the user to move the protective sheet in a direction of being away from the base unit housing to separate the protective sheet from the adhesive layer.

In addition, to accomplish the purposes described above, according to an embodiment of the present disclosure, an applicator assembly may comprise: an applicator body; a sensor unit including a sensor, a sensor unit housing to which the sensor is mounted, an adhesive layer provided at the sensor unit housing, and a protective sheet covering the adhesive layer; an insertion unit installed to the applicator body to move the sensor unit from a first position spaced away from the skin of the user to a second position where the sensor is inserted into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.

The applicator assembly according to an embodiment of the present disclosure may comprise a base unit including a base unit housing to which the sensor unit housing is coupled and an adhesive portion provided at the base unit housing to be attached to the skin of the user, and spaced apart from the sensor unit and separably coupled to the applicator body, and the sensor unit housing may be configured to be attachable to the base unit housing by the adhesive layer in the second position.

Additionally, to accomplish the purposes described above, according to an embodiment of the present disclosure, an applicator assembly may comprise: a sensor unit including a sensor and a sensor unit housing to which the sensor is mounted; a base unit including a base unit housing, an adhesive layer provided at one side of the base unit housing to attach the sensor unit housing and the base unit housing, a protective sheet covering the adhesive layer, and an adhesive portion provided at another side of the base unit housing to be attached to the skin of the user; an applicator body to which the base unit is separably coupled; an insertion unit mounted to the applicator body to insert the sensor into the skin of the user by moving the sensor unit from a first position spaced apart from the base unit to a second position where the sensor unit coupled with the base unit; and a moving tab to which a portion of the protective sheet is coupled and is movably installed to the applicator body to be movable by the user to move the protective sheet in a direction of being away from the base unit housing to separate the protective sheet from the adhesive layer.

### Advantageous Effects of Invention

According to the present disclosure, by manufacturing the sensor unit in an assembled state within the applicator, the additional work for a user to attach the sensor unit to the skin of a user is minimized, and the sensor unit can be attached to the skin of a user simply by operating the applicator.

Additionally, according to the present disclosure, by covering and protecting the adhesive layer of the sensor unit or the adhesive layer of the base unit with a protective sheet, it is possible to prevent a problem of deteriorating the adhesiveness of the adhesive layer before inserting the sensor into the skin of a user.

In addition, according to the present disclosure, the protective sheet that covers and protects the adhesive layer of the sensor unit or the adhesive layer of the base unit can be easily removed, and user convenience is excellent.

Additionally, according to the present disclosure, the insertion operation of the sensor can be performed after the moving tab for removing the protective sheet moves to remove the protective sheet, thereby preventing the issues of inadvertent malfunction by a user.

### BRIEF DESCRIPTION OF THE DRAWINGS

FIG. 1 is a perspective view showing an applicator assembly according to an embodiment of the present disclosure.
FIG. 2 shows a body attachable unit attached to the skin of a user.
FIG. 3 is a perspective view showing a body attachable unit.
FIGS. 4 and 5 are exploded perspective views showing a sensor unit.
FIG. 6 shows a needle coupled to a sensor unit.
FIG. 7 shows the base unit.
FIG. 8 is a cross-sectional view showing the sensor unit and the base unit before they are coupled.
FIG. 9 is a cross-sectional view showing a sensor unit and a base unit coupled to form a body attachable unit.
FIG. 10 is a cross-sectional view taken along line I-I in FIG. 1.
FIG. 11 is a cross-sectional view taken along line II-II in FIG. 1.
FIG. 12 is an exploded perspective view of the applicator according to an embodiment of the present disclosure.
FIG. 13 shows the locking hook and moving tab separated from the base frame.
FIG. 14 is a perspective view showing the bottom surface of the middle frame.
FIG. 15 shows an exploded view of the insertion unit assembly.
FIG. 16 shows the carrier and needle of the needle assembly separated.
FIG. 17 is a cross-sectional view showing the carrier and the needle of the needle assembly coupled
FIG. 18 is a perspective view showing a part of the applicator.
FIG. 19 shows the moving tab separated from the protective sheet.
FIG. 20 shows the process of removing the protective sheet of the base unit using a moving tab.
FIGS. 21 to 27 show the process of inserting the sensor of the sensor unit into the skin of a user using an applicator according to an embodiment of the present disclosure.
FIG. 28 is a cross-sectional view showing an applicator assembly according to another embodiment of the present disclosure.
FIG. 29 shows the protective sheet and moving tab of the applicator assembly shown in FIG. 28.
FIG. 30 is a cross-sectional view showing an applicator assembly according to another embodiment of the present disclosure.
FIG. 31 shows the sensor unit of the applicator assembly shown in FIG. 30.
FIG. 32 is a cross-sectional view showing an applicator assembly according to another embodiment of the present disclosure.
FIG. 33 shows the sensor unit of the applicator assembly shown in FIG. 32.
FIG. 34 is a cross-sectional view showing an applicator assembly according to another embodiment of the present disclosure.
FIG. 35 shows a state in which the protective sheet is removed using the moving tab of the applicator assembly shown in FIG. 34.
FIG. 36 is a cross-sectional view showing an applicator assembly according to another embodiment of the present disclosure.
FIG. 37 shows a state in which the protective sheet is removed using the moving tab of the applicator assembly shown in FIG. 36.
FIGS. 38 and 39 show other embodiments of the sensor unit and base unit.

### DETAILED DESCRIPTION OF EMBODIMENTS

Hereinafter, the applicator and applicator assembly for a transcutaneous sensor according to the present disclosure will be described in detail with reference to the drawings.

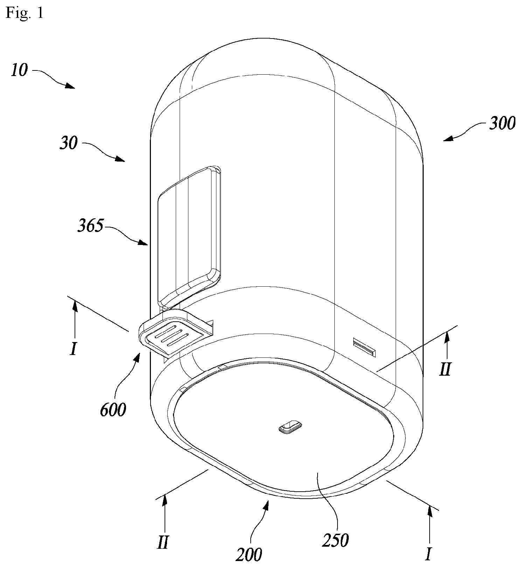

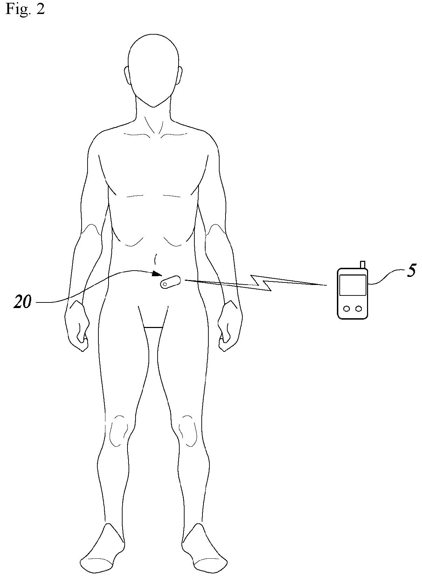

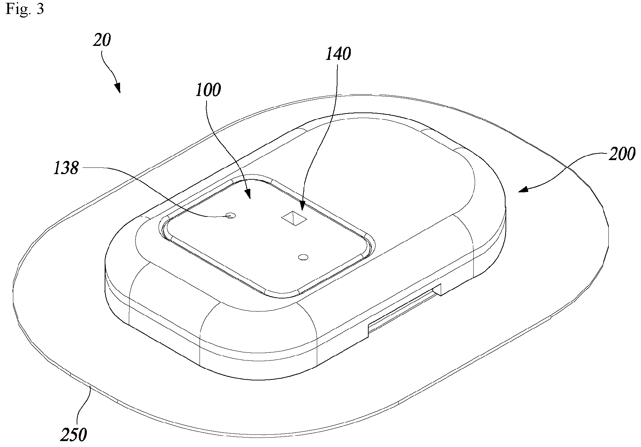

FIG. 1 is a perspective view showing an applicator assembly according to an embodiment of the present disclosure, FIG. 2 is a perspective view showing the body attachable unit attached to the skin of a user, and FIG. 3 is a perspective view showing the body attachable unit.

An applicator assembly (10) according to an embodiment of the present disclosure includes a sensor unit (100) including a sensor (110) that is inserted into the skin of a user and measures biometric information, and a base unit (200) that is coupled with the sensor unit (100) to form a body attachable unit (20), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

As shown in FIG. 2, the body attachable unit (20) can be attached to the skin of a user to measure biometric information and wirelessly transmit the measured data to the external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may measure the blood glucose level of a user and transmit the blood glucose measurement information to the external terminal (5).

As shown in FIG. 3, the body attachable unit (20) includes the sensor unit (100) for measuring the biometric information of a user, and the base unit (200) that is attached to the skin of a user and coupled to the sensor unit (100). do. The base unit (200) may have electronic components installed therein. This base unit (200) is electrically connected to the sensor unit (100) and can process biometric information measured by the sensor unit (100) and transmit it to the external terminal (5).

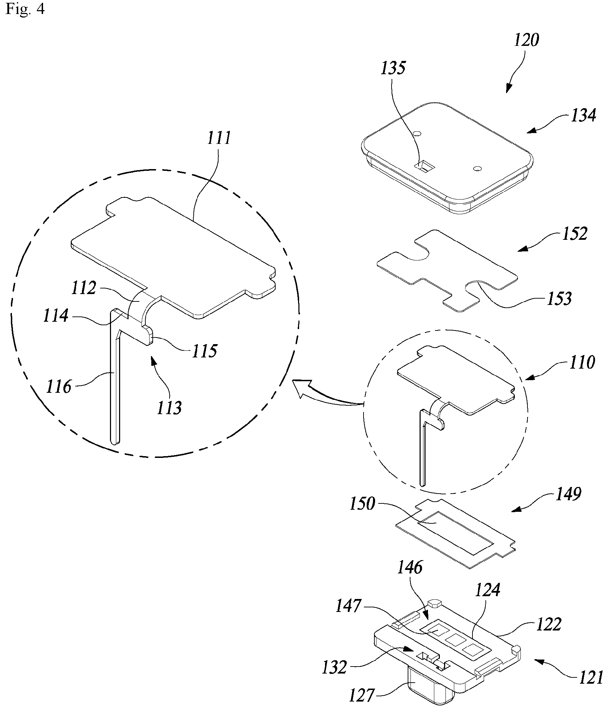

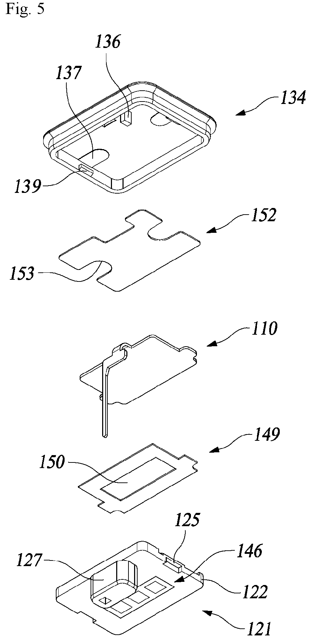

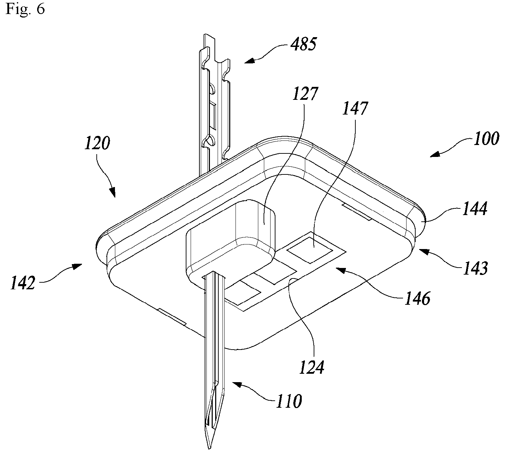

As shown in FIGS. 4 to 9, the sensor unit (100) includes the sensor (110) inserted into the skin of a user, a sensor unit housing (120) to which the sensor (110) is coupled, a sensor unit-electrical contact portion (146) electrically connected to the sensor (110) coupling to the sensor unit housing (120), a sensor adhesive portion (149) and an adhesive pad (152) for fixing the sensor (110) to the sensor unit housing (120).

The sensor (110) includes a sensor body (111), a connection portion (112) connected to one side of the sensor body (111), a middle portion (113) connected to the sensor body (111) through the connection portion (112), and an insertion portion (116) connected to the middle portion (113) so as to be inserted into the skin of a user. The sensor body (111) is disposed inside the sensor unit housing (120) so as to be in contact with the sensor unit-electrical contact portion (146). The sensor body (111) may be provided with an electrode electrically connected to the sensor unit-electrical contact portion (146). The connection portion (112) extends from the edge of the sensor body (111) and connects the sensor body (111) and the middle portion (113) to lie on different planes. The sensor (110) may be manufactured in a flat shape in which the sensor body (111) lies on the same plane as the middle portion (113) and the insertion portion (116), and then manufactured in a form in which the connection portion (112) is bent and deformed so that the sensor body (111) lies on a different plane from the middle portion (113) and the insertion portion (116).

The middle portion (113) and the insertion portion (116) are connected to the sensor body (111) so as to lie on a different plane from the sensor body (111). In the drawing, the middle portion (113) and the insertion portion (116) are shown to be disposed on the same plane, and the middle portion (113) is disposed perpendicular to the sensor body (111), but the angle between the middle portion (113) and the sensor body (111) or the angle between the insertion portion (116) and the sensor body (111) can be changed in various ways.

The middle portion (113) includes a first extension portion (114) that protrudes to one side of the connection portion (112), and a second extension portion (115) that protrudes from the connection portion (112) in a direction opposite to the direction in which the first extension portion (114) protrudes from the connection portion (112). The insertion portion (116) may be connected to the first extension portion (114) and inserted into the skin of a user. The insertion portion (116) has a relatively thin and long shape so as to be smoothly inserted into the skin of a user. The connection portion (112), the middle portion (113), and the insertion portion (116) may be provided with a conductive trace electrically connected to the electrode of the sensor body (111). The sensor (110) may be coupled to the sensor unit housing (120) so that the sensor body (111), the connection portion (112) and the middle portion (113) are located inside the sensor unit housing (120) and only the insertion portion (116) protrudes from the sensor unit housing (120).

The sensor unit housing (120) includes a housing base (121) and a housing cap (134) that covers the top of the housing base (121). A space is provided inside the sensor unit housing (120) to accommodate the sensor (110), and a housing opening (140) into which a needle (450) for inserting the sensor (110) into the skin of a user is inserted is formed in the middle of the sensor unit housing (120) to penetrate the sensor unit housing (120) in the thickness direction.

The housing base (121) includes a base portion (122) supporting the sensor body (111) and a boss (127) protruding from the surface of the base portion (122). On one side of the base portion (122), a through hole (124) is formed to penetrate the base portion (122). A sensor unit-electrical contact portion (146) is coupled to the through hole (124). A detent groove (125) is provided at the edge of the base portion (122). The boss (127) protrudes from one of the surfaces on both sides of the base portion (122) where the sensor body (111) is not placed. The boss (127) is provided with a housing base hole (132). The housing base hole (132) extends from the end of the boss (127) to one surface of the base portion (122). The insertion portion (116) of the sensor (110) and needle (485) may be inserted into the housing base hole (132).

The housing cap (134) is coupled to the housing base (121) and covers the sensor body (111) of the sensor (110) placed on the housing base (121) and the upper part of the housing base (121). A housing cap hole (135) is formed in the middle of the housing cap (134) to penetrate the housing cap (134) in the thickness direction. The housing cap hole (135) is connected to the housing base hole (132) of the housing base (121) to form the housing opening (140). The needle (450) for inserting the sensor (110) into the skin of a user is inserted along with the sensor (110) into the housing opening (140). A sensor unit groove (138) is formed on the upper surface of the housing cap (134). Additionally, the housing cap (134) is provided with a detent protrusion (139) corresponding to the detent groove (125) of the housing base (121). The housing cap (134) may be coupled to the housing base (121) by inserting the detent protrusion (138) into the detent groove (125) and engaging the housing base (121). A protrusion (137) and a retention protrusion (136) are provided on the inner surface of the housing cap (134) facing the housing base (121). The protrusion (137) and the retention protrusion (136) protrude toward the housing base (121). The protrusion (137) and the retention protrusion (136) may contact the sensor (110) to prevent movement of the sensor (110) and stably fix the sensor (110) to the sensor unit housing (120). The protrusion (137) may contact the sensor body (111) of the sensor (110) and press the sensor body (111) toward the housing base (121). Therefore, the sensor body (111) can remain firmly fixed to the housing base (121) without being lifted from the housing base (121).

The protrusion (137) may contact the sensor body (111) and press the sensor body (111) toward the housing base (121). Therefore, the sensor body (111) may remain firmly fixed to the housing base (121) without being lifted from the housing base (121). The retention protrusion (136) is inserted into the housing base hole (132) and comes into contact with the sensor (110). That is, the retention protrusion (136) may be arranged such that its end faces the second extension (115) of the middle portion (113) inserted into the housing base hole (132) to restrain the movement of the middle portion (113) so that the middle portion (113) of the sensor (110) is not lifted. As the action of the retention protrusion (136), the insertion portion (116) of the sensor (110) can be stably maintained in a protruded state from the sensor unit housing (120) by a certain length. And, due to the action of the retention protrusion (136), the insertion portion (116) of the sensor (110) may not retreat from the skin of a user while being inserted into the skin of a user, and remain inserted at a certain depth into the skin of a user. In addition, after the insertion portion (116) is inserted into the skin of a user with the needle 485, the retention protrusion (136) prevents the middle portion (113) from moving while the needle (485) exits the skin of a user, so that the insertion portion (116) cannot retreat in the same direction as the needle (485). Accordingly, it is possible to prevent a problem in which the insertion portion (116) inserted into the skin of a user moves in the direction of exiting the skin of a user in the process of the needle (485) coming out of the skin of a user. Additionally, the insertion portion (116) partially comes out of the skin, thereby preventing the measurement accuracy of the sensor (110) from being reduced.

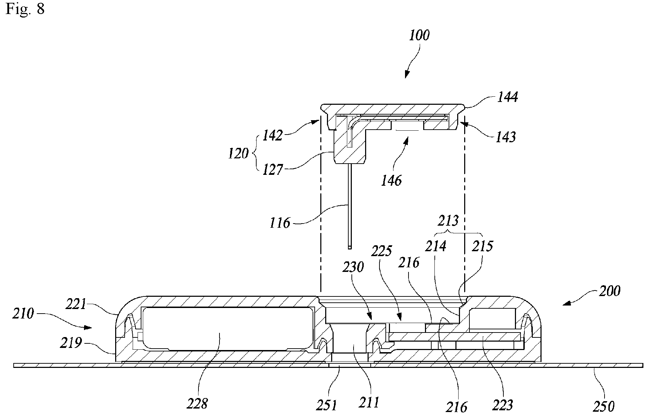

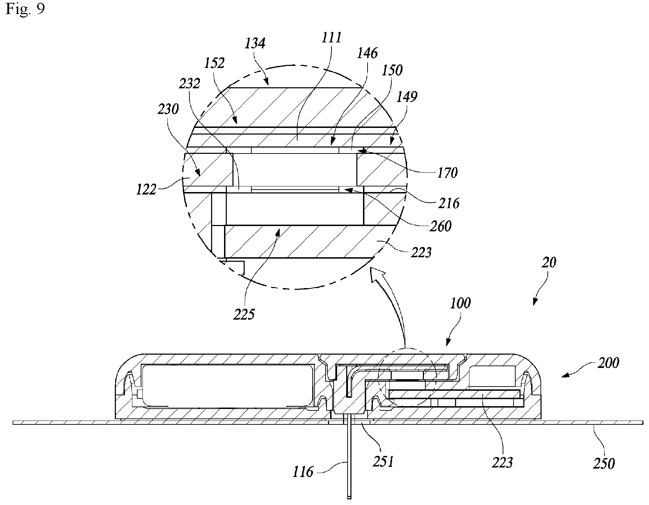

The housing cap (134) forms the housing body (142) together with the base portion (122). That is, the sensor unit housing (120) may be configured to include a housing body (142) and a boss (127) protruding from one side of the housing body (142). As shown in FIGS. 8 and 9, the housing body (142) is shaped to fit into a base unit recess (213) of the base unit (200). The housing body (142) includes a body portion (143) provided with a boss (127) and a cover portion (144) that is wider than the body portion (143).

The sensor unit housing (120) is not limited to the configuration shown, and may be changed to various other configurations on which the sensor (110) may be mounted and coupled to the base unit (200). That is, the sensor unit housing (120) includes a housing base (121) and the housing cap (134), but it can be changed to various configurations other than a configuration including a housing body (142) and a boss (127) protruding from the housing body (142).

The sensor unit-electrical contact portion (146) is disposed in the sensor unit housing (120) to be electrically connected to the sensor body (111). The sensor unit-electrical contact portion (146) is inserted into the through hole (124) of the base portion (122) and is disposed to vertically penetrate the base portion (122). Some part of the sensor unit-electrical contact portion (146) is exposed to one surface of the base portion (122) where the sensor body (111) is located, and another part is exposed to the other side of the base portion (122), so that the sensor (110) and a base unit-electrical contact portion (225) of the base unit (200) can be electrically connected.

The sensor adhesive portion (149) is disposed between the housing base (121) and the sensor body (111) to attach the sensor body (111) to the housing base (121). The sensor adhesive portion (149) has adhesive properties on both sides. That is, one side of the sensor adhesive portion (149) is adhered to the housing base (121), and the other surface of the sensor adhesive portion (149) is adhered to the sensor body (111). A sensor adhesive portion opening (150) is formed in the middle of the sensor adhesive portion (149) to penetrate the sensor adhesive portion (149) in the thickness direction. The sensor body (111) is in contact with the sensor unit-electrical contact portion (146) through the sensor adhesive portion opening (150). Therefore, the sensor adhesive portion (149) seals between the sensor body (111) and the sensor unit-electrical contact portion (146), thereby preventing moisture or foreign substances from entering an electrical connection portion (170) between the sensor body (111) and the sensor unit electrical contact portion (146).

The shape of the sensor adhesive portion (149) is not limited to that shown and can be changed in various ways.

The adhesive pad (152) is disposed between the housing cap (134) and the sensor body (111) to attach the sensor body (111) to the housing cap (134). The adhesive pad (152) has adhesive properties on both sides. That is, one side of the adhesive pad (152) is adhered to the housing cap (134), and the other side of the adhesive pad (152) is adhered to the sensor body (111). An adhesive pad groove (153) is formed in the adhesive pad (152) to penetrate the adhesive pad (152) in the thickness direction. The protrusion (137) of the housing cap (134) contacts the sensor body (111) through the adhesive pad groove (153), thereby pressing the sensor body (111) toward the housing base (121).

The shape of the adhesive pad (152) is not limited to that shown and can be changed in various ways.

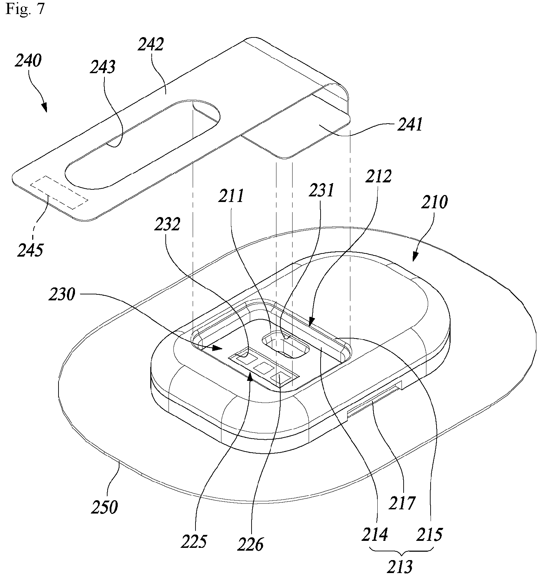

As shown in FIGS. 7 to 9, the base unit (200) includes a base unit housing (210) to which the sensor unit (100) is coupled, electronic components installed inside the base unit housing (210), an adhesive layer (230) provided on the base unit housing (210) to be attached to the sensor unit (100), and a protective sheet (240) covering the adhesive layer (230). The electronic component may include a circuit board (223), a base unit electrical contact portion (225) in contact with the sensor unit-electrical contact portion (146) of the sensor unit (100), a battery (228), etc. The circuit board (223) may be equipped with a processor chip for processing signals, a communication chip for wireless communication with the external terminal (5), etc.

The base unit housing (210) is provided with an insertion hole (211) through which the insertion portion (116) of the sensor (110) and the needle (450) can pass, and a mounting portion (212) in which the sensor unit housing (120) is coupled. The mounting portion (212) includes a base unit recess (213) and a supporting surface (216) provided inside the base unit recess (213). The insertion hole (211) is shaped so that the boss (127) of the sensor unit (100) can be fitted and is disposed inside the base unit recess (213). The supporting surface (216) may be flat so as to stably support the sensor unit housing (120) of the sensor unit (100). The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) can be made in a shape corresponding to the body portion (143) of the sensor unit housing (120), and the second recess (215) can be made in a shape corresponding to the cover portion (144) of the sensor unit housing (120). Therefore, the sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). A part of a locking hook (360) provided on the applicator (30) may be inserted into the housing groove (217).

The base unit-electrical contact portion (225) is arranged on the mounting portion (212) and contacts the sensor unit-electrical contact portion (146) of sensor unit (100) when sensor unit (100) is coupled to the base unit (200). The base unit-electrical contact portion (225) is electrically connected to the circuit board (223) and is installed in the base unit housing (210) so that a portion is exposed to the base unit recess (213). The base unit-electrical contact portion (225) may include a plurality of terminal portions (226) for transmitting electrical signals. The terminal portion (226) may electrically connect the sensor unit-electrical contact portion (146) and the circuit board (223) by contacting the terminal portion (147) of the sensor unit (100).

As shown, the base unit housing (210) may include a lower housing (219) and an upper housing (221) that covers the upper part of the lower housing (219), but its shape may be changed in various ways.

Adhesive layer (230) is disposed on the supporting surface (216). The adhesive layer (230) may be in the form of a double-sided tape with adhesive properties on both sides. One side of the adhesive layer (230) is adhered to the supporting surface (216), and the other side of the adhesive layer (230) is covered with a protective sheet (240). The adhesive layer (230) may be attached to the sensor unit (100) after the protective sheet (240) is separated. In the middle of the adhesive layer (230), an adhesive layer hole (231) and an adhesive layer opening (232) are formed to penetrate the adhesive layer (230) in the thickness direction. The adhesive layer hole (231) is connected to the insertion hole (211). The boss (127) of the sensor unit (100) may pass through the adhesive layer hole (231) and be inserted into the insertion hole (211). The adhesion layer opening (232) exposes base unit-electrical contact portion (225) to the outside of the adhesive layer (230). The base unit-electrical contact portion (225) may contact sensor unit-electrical contact portion (146) of the sensor unit (100) through adhesive layer opening (232). Therefore, the adhesive layer (230) seals between the sensor unit-electrical contact portion (146) and the base unit-electrical contact portion (225), thereby preventing moisture or foreign substances from entering the electrical connection portion (260) between the sensor unit (100) and the base unit (200).

The shape of the adhesive layer (230) is not limited to that shown and can be changed in various ways.

The protective sheet (240) covers and protects the adhesive layer (230). The protective sheet (240) is made of a material that is detachably attached to the adhesive layer (230). If the adhesive layer (230) is left exposed to the air for a long time, the adhesiveness of the adhesive layer (230) may be reduced. The protective sheet (240) covers the adhesive layer (230) to prevent the problem of poor adhesiveness of the adhesive layer (230), and makes it easier for operators to handle the base unit (200) during the manufacturing process of the base unit (200) or the process of assembling the base unit (200) to the applicator (30). The protective sheet (240) includes a protection portion (241) adhered to the adhesive layer (230) and a wing portion (242) extending from an edge of the protection portion (241). A protective sheet hole (243) is formed in the wing portion (242) to penetrate the wing portion (242) in the thickness direction. The wing portion (242) may extend by a certain length from the edge of the base unit housing (210) and be coupled to the moving tab (600) of the applicator (30).

The shape of the protective sheet (240) is not limited to that shown and can be changed in various ways.

An adhesive member (245) is provided on one side of the protective sheet (240). The adhesive member (245) may be in the form of a double-sided tape with adhesive properties on both sides. One side of the adhesive member (245) may be adhered to the protective sheet (240), and the other side of the adhesive member (245) may be adhered to the moving tab (600). Therefore, the protective sheet (240) can be coupled to the moving tab (600) through the adhesive member (245).

The shape, number, and location of the adhesive members (245) are not limited to those shown and can be changed in various ways.

The base unit (200) is mounted on the applicator (30) in a state in which the protective sheet (240) is attached to the adhesive layer (230). The protective sheet (240) may be separated from the adhesive layer (230) by the moving tab (600) of the applicator (30) before the sensor unit (100) is coupled to the base unit (200). After the protective sheet (240) is removed from the base unit (200) by the moving tab (600), the sensor unit (100) may move toward the base unit (200) and be coupled to the base unit (200).

An adhesive portion (250) is provided on the surface of the base unit housing (210). The adhesive portion (250) is attached to the surface of the lower housing (219) to adhere the base unit housing (210) to the skin of a user. In the middle of the adhesive portion (250), an adhesive hole (231) through which the insertion portion (116) of the sensor (110) and the needle (450) can pass is formed to penetrate the adhesive portion (250) in the thickness direction.

The adhesive portion (250) may be covered and protected with a protective sheet. The protective sheet covering the adhesive portion (250) may be removed during the process of attaching the base unit (200) to the skin of a user.

The sensor unit (100) and the base unit (200) are installed in the applicator (30) in a separated state, and are coupled to each other in the process of operating the applicator (30) to insert the sensor (110) into the skin of a user, forming the body attachable unit (20). The sensor unit (100) may be mounted on the applicator (30) while the needle (450) coupled, and moved toward the base unit (200) with the needle (450) coupled thereto to be coupled to the base unit (200). After the sensor unit (100) is coupled to the base unit (200), the needle (450) is separated from the sensor unit (100), and only the body attachable unit (20) remains on the skin of a user.

As another exemplary embodiment, the base unit (200) may take a configuration that simply supports the sensor unit (100) so as not to be separated from the skin of a user, without electronic components. In this case, a separate electronic unit that can process biometric information measured by the sensor unit (100) and transmit it to the external terminal (5) may be detachably coupled to the base unit (200). A separate electronic unit may be coupled to the base unit (200) to be electrically connected to the sensor unit (100) after the sensor unit (100) is coupled to the base unit (200).

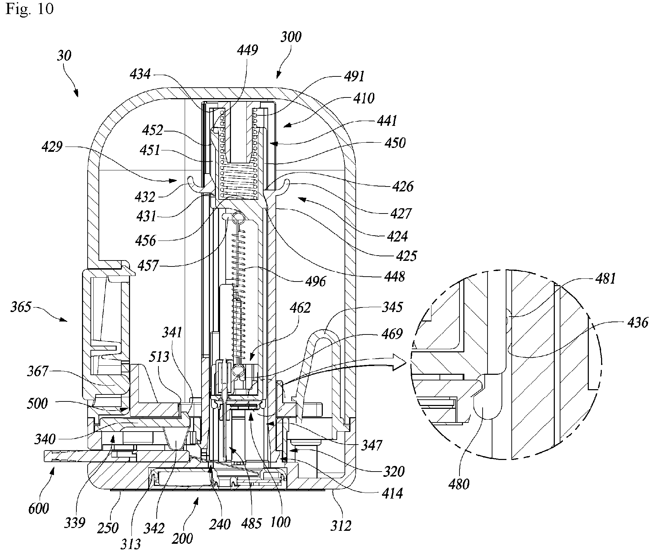

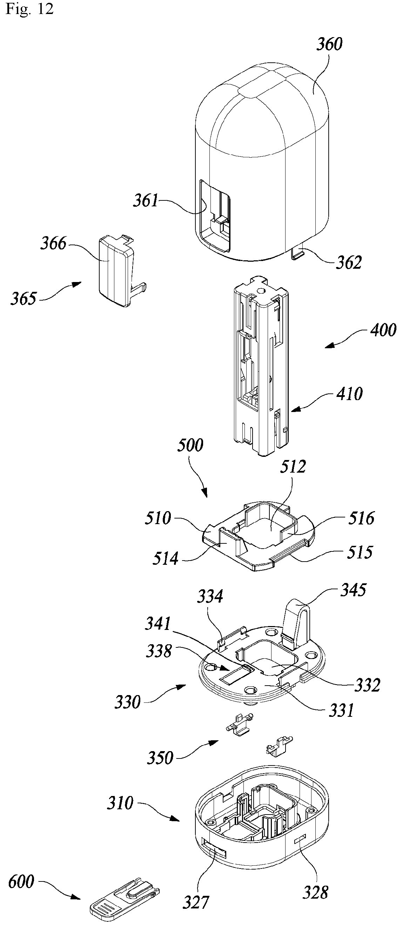

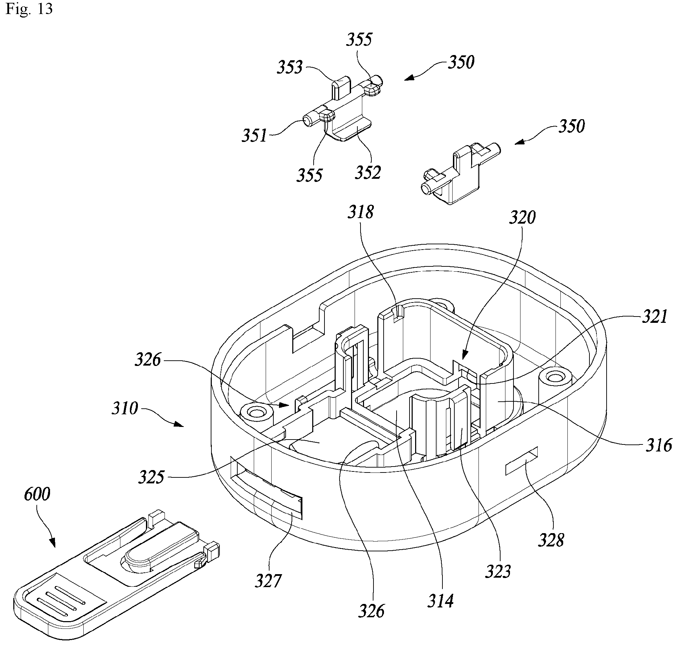

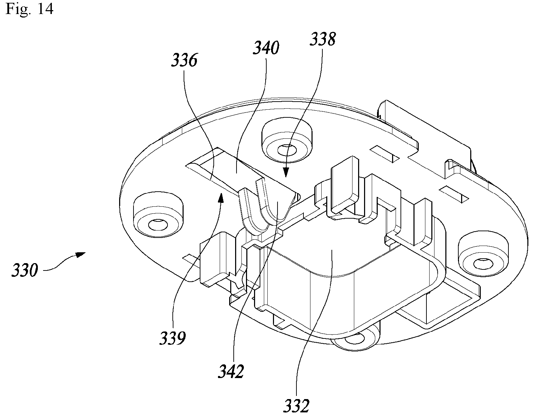

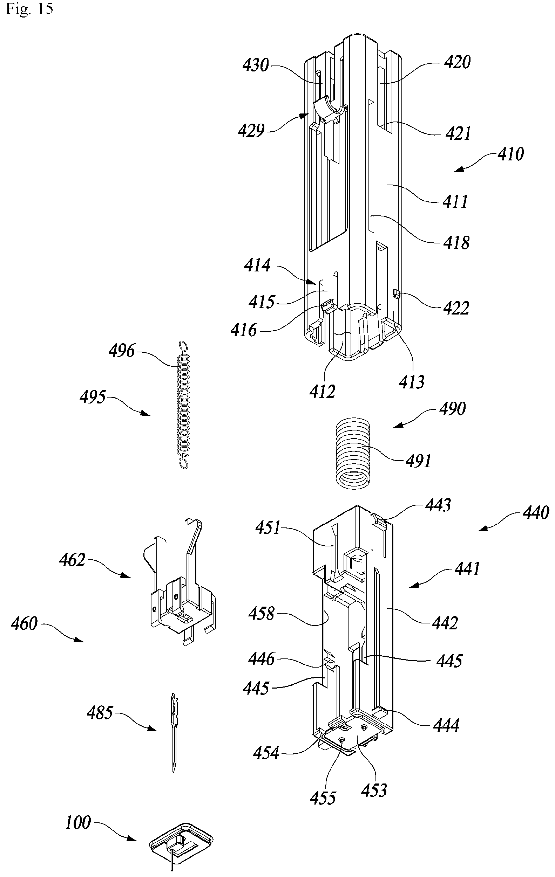

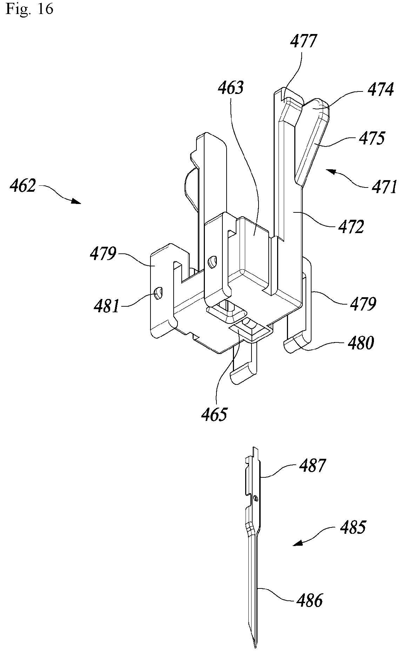

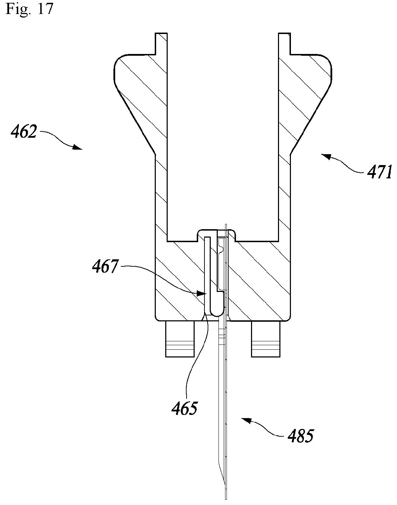

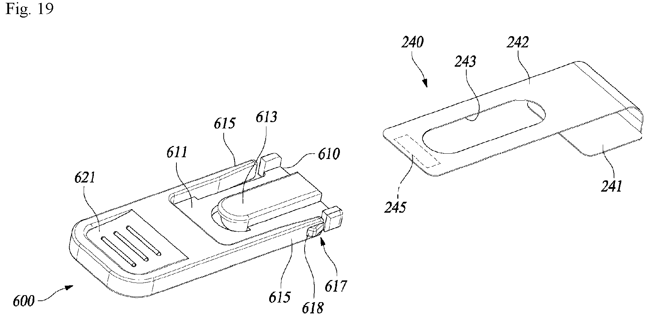

Referring to FIGS. 10 to 20, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (250).

The applicator (30) includes an applicator body (300) to which the base unit (200) is detachably coupled, an operating member (365) installed on the applicator body (300) for user manipulation, an insertion unit assembly (400) including an insertion unit (440) for inserting the sensor (110) into the skin of a user, a stopper member (500) movably installed on the applicator body (300), and a moving tab (600) for removing the protective sheet (240) of the base unit (200). The insertion unit (400) may move the sensor unit (100) from a first position spaced apart from the base unit (200) by a preset distance to a second position coupled with the base unit (200). The sensor unit (100) and a top case (350) which is coupled to the middle frame (330) and covers the upper part of the middle frame (330).

The applicator body (300) includes a base frame (310), a middle frame (330) disposed on the base frame (310), and a top case (360) that is coupled to the middle frame (330) and covers the upper part of the middle frame (330).

The base frame (310) has a bottom portion (312) that can be in contact with the skin of a user. The bottom portion (312) of the base frame (310) is provided with a recess (313) on which the base unit (200) is mounted. The base unit (200) is detachably coupled to the recess (313) so that the adhesive portion (250) can face the skin of a user and placed in a second position spaced apart from the sensor unit (100). A base frame opening (314) is formed in the middle of the frame base portion (311) to penetrate the frame base portion (311) in the thickness direction. A fence portion (316) is provided to protrude around the base frame opening (314). When the base frame (310) is coupled to the middle frame (330), the fence portion (316) is inserted into the middle frame opening (334) of the middle frame (330). The fence portion (316) may support the column member (410) in contact with the outer surface of the column member (410) constituting the insertion unit assembly (400). A coupling groove (318) is formed on one side of the fence portion (316). Additionally, an applicator body snap portion (320) for fixing the column member (410) is provided on one side of the base frame (310). The applicator body snap portion (320) includes a snap groove (321) with which the column member (410) can be engaged. A support portion (326) and an elastic arm (323) are provided on both sides of the base frame opening (314). The support portion (326) may rotatably support the locking hook (350) for temporarily fixing the base unit (200) to the applicator body (300). The elastic arm (323) can elastically support the locking hook (350).

The locking hook (350) may engage the base unit (200) mounted in the recess (313) and secure the base unit (200) without being separated from the recess (313). The locking hook (350) may be disengaged from the base unit (200) after the body attachable unit (20) is attached to the skin of a user. The locking hook (350) is pivotally coupled to the support portion (326). The locking hook (350) includes a pivot portion (351) rotatably coupled to the support portion (326), a hook portion (352) connected to the pivot portion (351), and a rod (353) and a lever portion (355) each protruding from the pivot portion (351). The hook portion (352) may be inserted into the housing groove (217) of the base unit (200) and engaged with the base unit (200). The hook portion (352) protrudes from one side of the pivot portion (351), and the rod (353) protrudes from the other side of the pivot portion (351). A part of the hook portion (352) is located in the recess (313) of the base frame (310). The lever portion (355) protrudes from the pivot portion (351) toward the base frame opening (314). The locking hook (350) rotates around the pivot portion (351) while elastically deforming the elastic arm (323) when the lever portion (355) is pressed by the shuttle (441) of the insertion unit (440), thereby being disengaged from the base unit (200).

The elastic arm (323) may contact the locking hook (350) and apply elastic force to the locking hook (350) in a direction that engages the base unit (200). That is, the elastic arm (323) can elastically support the locking hook (350) so that the locking hook (350) remains engaged with the base unit (200). In the drawing, it is shown that the elastic arm (323) can apply elastic force to the rod (353) in a direction away from the recess (313), but the installation position of the elastic arm (323) or the position where the elastic arm (323) contacts the locking hook (350) may be changed in various ways.

A more specific operation of the locking hook (350) will be described later.

A stage (325) is provided on an inner side of the base frame (310). The stage (325) supports the moving tab (600) so that the moving tab (600) can move linearly stably. Applicator body detents (326) that can engage with the moving tab (600) are provided on both sides of the stage (325).

In addition, an entrance (327) and a coupling hole (328) are formed on the outer surface of the base frame (310). The moving tab (600) placed on the stage (325) may be pulled out to the outside of the base frame (310) through the entrance (327).

The specific configuration of the base frame (310) is not limited to that shown and can be changed in various ways.

The middle frame (330) includes a stage (331) supporting the stopper member (500). A middle frame opening (332) is provided in the middle of the stage (331) into which the fence portion (316) of the base frame (310) is inserted. The middle frame opening (332) is formed to correspond to the base frame opening (314) of the base frame (310). That is, when the base frame (310) and the middle frame (330) are coupled, the middle frame opening (332) and the base frame opening (314) are connected. The middle frame opening (332) is connected to the base frame opening (314) to form an applicator body groove (347). The column member (410) of the insertion unit assembly (400) is inserted into the applicator body groove (347) so that the insertion unit assembly (400) can be coupled to the applicator body (300). A guide protrusion (334) is provided at the edge of the stage (331). The guide protrusion (334) serves to guide the stopper member (500) to move linearly.

A locking unit (338) capable of restraining the movement of the stopper member (500) and a return member (345) capable of returning the stopper member (500) to its original position are installed in the middle frame (330).

The locking unit (338) is placed in the through hole (336) formed in the middle of the stage (331). The locking unit (338) includes a locking body (339) connected to the middle frame (330) so as to be engaged with the stopper member (500) or disengaged from the stopper member (500). The locking body (339) includes a body portion (340) elastically deformably connected to the middle frame (330), a hook portion (341) connected to one side of the body portion (340) so as to be engaged with the stopper member (500), and a leg portion (342) connected to the other side of the body portion (340) so as to come into contact with the moving tab (600). The locking body (339) may be biased in a direction in which the hook portion (341) engages the stopper member (500) when the leg portion (342) contacts the moving tab (600). Additionally, the locking body (339) may be biased in a direction in which the hook portion (341) is disengaged from the stopper member (500) when the leg portion (342) leaves the moving tab (600). That is, the locking body (339) can maintain a position in which the hook portion (341) does not protrude above the stage (331) and does not engage with the stopper member (500) when no other external force is applied. And, the locking body (339) is elastically deformed so that the hook portion (341) protrudes above the stage (331) when the moving tab (600) contacts the leg portion (342). At this time, the hook portion (341) may engage with the stopper member (500) and restrain the movement of the stopper member (500).

The configuration of the locking unit (338) is not limited to that shown and may be changed in various ways. As another exemplary embodiment, the locking unit may include a different type of locking body that is engaged with the stopper member (500) by being coupled to the applicator body (300) in a changeable angle. As another exemplary embodiment, the locking unit may include another type of locking body which is elastically supported on the applicator body (300). Additionally, the locking unit (338) may be installed in other locations of the applicator body (300) other than the middle frame (330).

The return member (345) can be elastically deformed by the stopper member (500) and can return the stopper member (500) moved by the operating member (365) to its original state. The return member (345) is elastically deformed by the stopper member (500) which is moved forward by the operating member (365), and can apply an elastic force to the stopper member (500) in a retreating direction.

The configuration of the return member (345) is not limited to that shown and may be changed in various ways. Additionally, the return member (345) may be installed in other locations of the applicator body (300) other than the middle frame (330).

The top case (360) covers the upper part of the middle frame (330). One side of the top case (360) is provided with a top case opening (361) where an operating member (365) is installed. The top case (360) has a hook portion (362) and can be coupled to the base frame (310) in such a way that the hook portion (362) engages the coupling hole (328) of the base frame (310).

The specific configuration of the top case (360) is not limited to that shown and can be changed in various ways. Additionally, the method of coupling the top case (360) and the base frame (310), or the method of coupling the top case (360) and the middle frame (330), may be changed in various ways.

In addition to the applicator body (300) including a base frame (310), a middle frame (330), and a top case (360) as shown, it can be changed to various other configurations in which it is coupled to the insertion unit assembly (400) to support the insertion unit assembly (400), and the base unit (200) can be detachably coupled. Additionally, the applicator body (300) may be coupled to the base unit (200) in various other ways other than using a locking hook.

The operating member (365) includes a button portion (366) and a pressing protrusion (367) protruding from the button portion (366). The operating member (365) is installed in the top case opening (361) of the top case (360) so as to be operated by a user. The operating member (365) may operate to move the stopper member (500). When the operating member (365) is pressed into the applicator body (300) by a user, the pressing protrusion (367) can move the stopper member (500) by pushing the stopper member (500) toward the return member (345).

In addition to the configuration shown, the operating member (365) may be changed to another configuration that can be operated by a user to move the stopper member (500). Additionally, the operating member (365) may be installed in other locations of the applicator body (300) other than the top case (360).

The insertion unit assembly (400) includes a column member (410) fixed to the applicator body (300) and an insertion unit (440) that is supported on the column member (410) to be coupled with the sensor unit (100) and moves the sensor unit (100) from the first position to the second position. The insertion unit (440) is coupled to the applicator body (300) through the column member (410). The insertion unit (440) includes a shuttle (441) movably installed inside the column member (410), and a needle assembly (460) that can move together with the shuttle (441). The shuttle (441) is coupled with the sensor unit (100) and can move from the first position to the second position inside the applicator body (300). The needle assembly (460) includes a carrier (462) that can relatively move with respect to the shuttle (441) and a needle (485) that is coupled to the carrier (462) and can be inserted into the skin of a user.

The column member (410) is fixed to the applicator body (300) and supports the insertion unit (440). The column member (410) includes a column member body (411). The column member body (411) has a space inside to accommodate the insertion unit (440), and has a column member opening (412) opened to the outside at one end. A plurality of column member legs (413) are provided at one end portion of the column member body (411) in a shape surrounding the column member opening (412). The column member leg (413) may be provided in an elastically deformable form. The column member (410) may be coupled to the applicator body (300) in a manner in which the column member leg (413) is inserted into the applicator body groove (343) of the applicator body (300).

Additionally, one end of the column member body (411) is provided with a column member snap portion (414) that can engage with the applicator body snap portion (320) of the applicator body (300). The column member (410) can be more firmly fixed to the applicator body (300) by engaging the column member snap portion (414) with the applicator body snap portion (320). The column member snap portion (414) includes an elastically deformable snap arm (415). The snap arm (415) is provided with a snap hook (416) that engages the snap groove (321) of the applicator body snap portion (320). When the column member (410) is inserted into the applicator body groove (347), a portion of the snap hook (416) is inserted into the snap groove (321). As the column member snap portion (414) engages with the applicator body snap portion (320), the column member (410) can be stably fixed to the applicator body (300). In addition, when the column member (410) is inserted into the applicator body groove (347), the fence portion (316) of the applicator body (300) is in close contact with the outer surface of the column member (410) to support the column member (410) more stably. In addition, when the column member (410) is inserted into the applicator body groove (347), by inserting the coupling protrusion (422) provided on one side of the column member leg (413) into the coupling groove (318) of the base frame (310), the fixing force for the column member (410) can be further increased.

In this way, the column member (410) can be simply coupled to the applicator body (300) by inserting one end into the applicator body groove (347) of the applicator body (300).

The coupling method of the applicator body (300) and the column member (410) is not limited to that shown and may be changed in various ways. As another exemplary embodiment, the position or number of applicator body snap portion (320) provided in the applicator body (300) and the position or number of column member snap portion (414) provided in the column member (410) can be changed in various ways. Additionally, a snap hook may be provided in the applicator body snap portion (320) and a snap groove may be provided in the column member snap portion (414). As another exemplary embodiment, the column member (410) may be coupled to the applicator body (300) by forced press-fitting into the applicator body groove (347). As another exemplary embodiment, the column member (410) may be coupled to the applicator body (300) in such a way that a portion of the applicator body (300) is inserted into the column member opening (412) of the column member (410).

Slits (418) are formed on both sides of the column member body (411) in a direction parallel to the moving direction of the shuttle (441). A release portion (419) is provided in the middle of the slit (418). The release portion (419) is a part of the carrier (462) that can be contacted while the carrier (462) moves toward the base unit (200). Due to the action of the release portion (419), the carrier (462) is disengaged from the shuttle (441) and can relatively move with respect to the shuttle (441). The specific operation of the release unit (419) will be described later.

Additionally, guide grooves (420) are formed on both sides of the column member body (411) in a direction parallel to the moving direction of the shuttle (441). The shuttle hook (443) of the shuttle (441) is placed on the guide groove (420), so that the shuttle hook (443) can move along the guide groove (420). A column member detent (421) is provided at the end of the guide groove (420) to limit the moving distance of the shuttle (441). When the shuttle (441) moves from the first position with the sensor unit (100) and reaches the second position, the shuttle hook (443) contacts the column member detent (421) so that the shuttle (441) can stop at the second position.

A clamping portion (424) and a blocking portion (429) that can restrict the movement of the shuttle (441) are provided on both sides of the column member body (411).

The clamping portion (424) may engage with the shuttle (441) and may be used to temporarily fix the shuttle (441) to the column member (410) during the process of assembling the insertion unit assembly (400). The clamping portion (424) includes a clamping arm (425) provided with a clamping portion detent (426) and a knob (427) connected to the clamping arm (425) for user manipulation. The clamping portion detent (426) may be formed to protrude inward from the column member body (411), or may be formed to engage with one side of the shuttle (441). The clamping arm (425) is elastically deformably connected to the column member body (411). When a user pulls the knob (427), the clamping arm (425) may be elastically deformed.

The insertion unit assembly (400) may be coupled to the applicator body (300) in a state in which the shuttle (441) is temporarily fixed to the column member (410) by the clamping portion (424). While the shuttle (441) is temporarily fixed to the column member (410) by the clamping portion (424), the shuttle (441) cannot move. When the insertion unit assembly (400) is coupled to the applicator body (300) in a state in which the shuttle (441) is temporarily fixed to the column member (410), the insertion unit (440) does not operate even if a user presses the operating member (365). Therefore, in order for the applicator (30) to be in an operable state, it is necessary to couple the insertion unit assembly (400) to the applicator body (300) and release the temporarily fixing state of the shuttle (441). In the process of assembling the insertion unit assembly (400), after the shuttle (441) is inserted into the interior of the column member (410), the shuttle (441) is temporarily fixed to the column member (410), when the clamping portion detent (426) of the clamping portion (424) engages with the shuttle latch (448) of the shuttle (441), the shuttle (441) is temporarily fixed to the column member (410). Since the shuttle latch (448) is engaged with the clamping portion detent (426), the shuttle (441) cannot relatively move with respect to the column member (410).

There are various advantages to temporarily fixing the shuttle (441) to the column member (410) during the process of assembling the insertion unit assembly (400). That is, if the shuttle (441) is temporarily fixed to the column member (410), it is possible to prevent the shuttle (441) from accidentally moving during the process of assembling the insertion unit assembly (400) or coupling the insertion unit assembly (400) to the applicator body (300). Additionally, since a sharp needle (485) is coupled to the shuttle (441), the risk of a safety accident caused by accidental movement of the needle (485) during the manufacturing process can be reduced.

After coupling the insertion unit assembly (400) to the applicator body (300), a user can pull the knob (427) to elastically deform the clamping arm (425), thereby disengaging the clamping portion detent (426) from the shuttle latch (448).

A variety of other methods may be used to temporarily fix the shuttle (441) to the column member (410) in addition to using the clamping portion (424) as shown. As another exemplary embodiment, a clamping portion may be provided on the shuttle (441) to engage with the column member (410). Additionally, it is possible to temporarily fix the shuttle (441) to the column member (410) using various other types of components in addition to the elastically deformable clamping arm.

The blocking portion (429) is for fixing the shuttle (441), which has reached the second position, to the second position. The blocking portion (429) includes a blocking portion arm (430) provided with a blocking portion detent (431) and a knob (432) connected to the blocking portion arm (430) for user manipulation. The blocking portion detent (431) may have a shape that protrudes inward from the column member body (411), or may have another shape that can be in contact with one side of the shuttle (441). The blocking portion arm (430) is elastically deformably connected to the column member body (411). After the applicator (30) operates to insert the sensor (110) into the skin of a user, the applicator (30) needs to be separated from the body attachable unit (20) and discarded. Since the needle (485) of the applicator (30) is inserted into the skin of a user and then removed from the skin of a user in the process of inserting the sensor (110) into the skin of a user, the needle (485) is contaminated after use of the applicator (30). Additionally, after using the applicator (30), when the shuttle (441) is moved to the first position and reloaded, the needle (485) may move accidentally, which may lead to a safety accident. The blocking portion (429) can prevent the above problem by restricting the movement of the shuttle (441) after the shuttle (441) moves to the second position. After the shuttle (441) moves to the second position, the blocking portion detent (431) comes into contact with one side of the shuttle (441), thereby preventing the shuttle (441) from moving to the first position. And, since the shuttle (441) is fixed in the second position after use of the applicator (30), the applicator (30) cannot be reused.

A retention portion (434) is provided inside the column member (410). The retention portion (434) serves to support the shuttle driver (490), which provides moving force to the shuttle (441).

The shuttle (441) is installed to be linearly movable inside the column member (410). In a state in which the column member (410) is coupled to the applicator body (300), the shuttle (441) can move from the first position to the second position with the sensor unit (100). The shuttle (441) includes a shuttle body (442) with a space inside to accommodate the needle assembly (460). A shuttle hook (443) is provided on one side of the shuttle body (442) to limit the movement range of the shuttle (441). The shuttle hook (443) protrudes from the shuttle body (442) and is inserted into the guide groove (420) of the column member (410), and when the shuttle (441) moves to the second position, the movement of the shuttle (441) may be restricted by contacting the column member detent (421) of the column member (410). A shuttle protrusion (444) is provided on the other side of the shuttle (441). The shuttle protrusion (444) protrudes from the shuttle body (442) so as to contact the stopper member (500). The shuttle (441) is fixed to the first position by contacting the shuttle protrusion (444) with the stopper member (500), and can move to the second position when the shuttle protrusion (444) leaves the stopper member (500). The shuttle (441) can move the locking hook (350) through the shuttle protrusion (444). That is, when the shuttle (441) reaches the second position, the shuttle protrusion (444) may press the lever portion (355) of the locking hook (350), so that the locking hook (350) may rotates and be disengaged from the base unit (200).

A pair of slits (445) are formed on both sides of the shuttle body (442) in a direction parallel to the moving direction of the shuttle (441). A shuttle detent (446) to which the carrier latch (477) of the carrier (462) can be engaged is provided at a middle portion of the slit (445). In a state in which the carrier latch (477) is engaged with the shuttle detent (1446), the carrier (162) moves with the shuttle (441) and cannot relatively move with respect to the shuttle (1441). And when the carrier latch (477) is disengaged from the shuttle detent (446), the carrier (462) can relatively move with respect to the shuttle (441).

On both sides of the shuttle body (442), a shuttle latch (448) that can engage with the clamping portion (424) of the column member (410), and a detent portion (449) that can contact to the blocking portion (429) are provided.

When the shuttle latch (448) engages with the clamping portion detent (426) of the clamping portion (424), the shuttle (441) may be temporarily fixed to the column member (410). When the temporary fixing state by the clamping portion (424) is released, the clamping portion detent (426) is inserted into the guide groove (450) formed in the shuttle body (442) to be adjacent to the shuttle latch (448). When the shuttle (441) moves from the first position to the second position, the clamping portion detent (426) may relatively move with respect to the shuttle (441) in the guide groove 1450. The shape or location of the shuttle latch (448) is not limited to that shown and may be changed in various ways.

The detent portion (449) is provided at the end of the shuttle body (442) so as to contact the blocking portion detent (431) of the blocking portion (429). After the shuttle (441) moves to the second position, the detent portion (449) is placed to face the blocking portion detent (41), so that the shuttle (441) cannot move to the first position. The blocking portion detent (1431) is inserted into the guide groove (451) formed in the shuttle body (442) and can relatively move with respect to the shuttle (441). The guide groove (451) is formed in a direction parallel to the moving direction of the shuttle (441). A shuttle inclined portion (452) is positioned on one side of the guide groove (451). The shuttle inclined portion (452) is provided in an inclined form with respect to the moving direction of the shuttle (441). When the shuttle (441) moves to the second position, the blocking portion detent (431) relatively moves with respect to the guide groove (451), and when it reaches the shuttle inclined portion (452), the blocking portion arm (430) may be elastically deformed. As the blocking portion arm (430) is elastically deformed, the blocking portion detent (431) can smoothly pass through the shuttle inclined portion (452) and reach a position facing the detent portion (449). The shape or location of the detent portion (449) is not limited to that shown and may be changed in various ways.

A shuttle bottom portion (453) in contact with the sensor unit (100) is provided at one end of the shuttle body (442). The shuttle bottom portion (453) is provided with a shuttle through-hole (454) and a pressing protrusion (455). A needle (485) movable inside the shuttle body (442) may protrude to the outside of the shuttle body (442) through the shuttle through-hole (454). The pressing protrusion (455) is provided to be inserted into the sensor unit groove (138) of the sensor unit (100). When the sensor unit (100) is mounted on the insertion unit (440), the pressing protrusion (455) is inserted into the sensor unit groove (138) of the sensor unit (100), so that the sensor unit (100) can maintain its fixed position without moving.

A retention portion (456) and a fixing portion (457) are provided inside the shuttle (441). The retention portion (456) supports the shuttle driver (490). A needle release driver (495) that provides a moving force to the carrier (462) is coupled to the fixing portion (457).

In addition, the shuttle (441) includes a shuttle opening (458). The shuttle opening (458) is formed on one side of the shuttle body (442) to open outward. In the process of assembling the insertion unit assembly (400), the needle assembly (460) may be installed inside the shuttle body (442) through the shuttle opening (458).

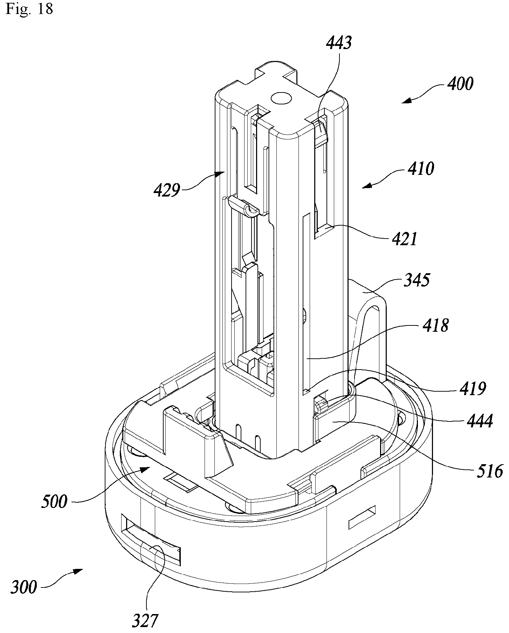

The shuttle (441) moves by receiving movement force from the shuttle driver (490). The shuttle driver (490) includes an elastic member (491) that applies elastic force to the shuttle (441) in a direction in which it moves from the first position to the second position. The elastic member (491) has one end in contact with the retention portion (434) of the column member (410) and the other end in contact with the retention portion (456) of the shuttle (441). The shuttle (441) may maintain a state in which the elastic member (491) is elastically deformed and temporarily fixed to the column member (410) by the clamping portion (424). And after the insertion unit assembly (400) is coupled to the applicator body (300), when the clamping portion (424) is released from the shuttle latch (448), the shuttle protrusion (444) contacts the stopper member (500), and the movement of the shuttle (441) is restricted by the stopper member (500). As shown in FIG. 18, when the stopper member (500) comes into contact with the shuttle protrusion (444) and supports the shuttle protrusion (444), the shuttle (441) remains stopped at the first position. And, when the stopper member (500) is moved by the operating member (365) and leaves the shuttle protrusion (444), the shuttle (441) can move to the second position by the elastic force of the elastic member (491).

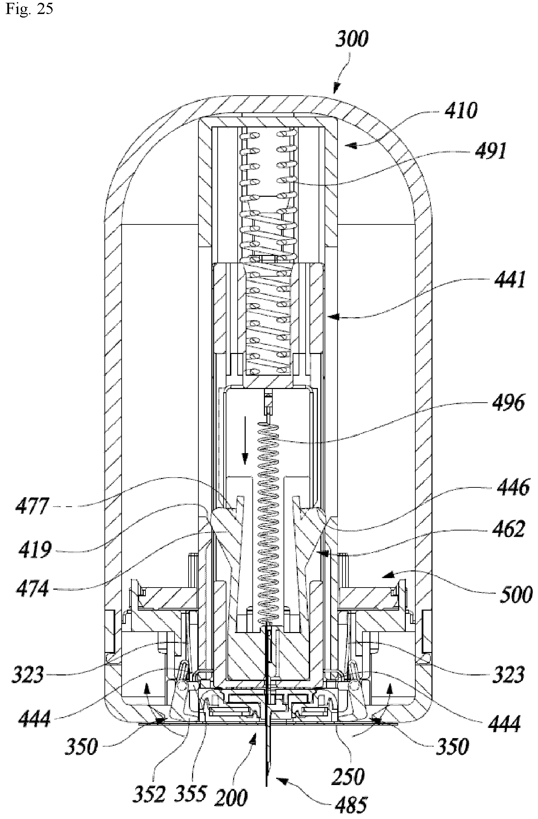

The shuttle (441) may move the sensor unit (100) toward the base unit (200) by moving from the first position to the second position, and release the locking hook (350) when it reaches the second position. As shown in FIG. 25, when the shuttle (441) reaches the second position, the shuttle protrusion (444) comes into contact with the lever portion (365) of the locking hook (360) and presses the lever portion (355). At this time, the locking hook (350) rotates around the pivot portion (351) while biasing the elastic arm (323) of the applicator body (300), so that the hook portion (352) deviates from the base unit (200). Accordingly, the base unit (200) may be disengaged from the locking hook (350) and separated from the applicator body (300).

The shuttle driver (490) may be changed to various other configurations that can provide a moving force to the shuttle (441) in addition to the configuration including the elastic member (491) in the form of a coil spring.

The needle assembly (460) includes a carrier (462) to which the sensor unit (100) is detachably coupled, and a needle (485) coupled to the carrier (462) to penetrate the sensor unit (100). The needle (485) may move forward from the first position to the second position in a state coupled with the sensor unit (100) to be inserted into the skin of a user, and may be pulled back together with the carrier (462) at the second position to be separated from the skin of a user and the sensor unit (100).

The carrier (462) is coupled to the shuttle (441) to be relatively movable. A portion of the carrier (462) engages with the shuttle (441) so that it can advance together with the shuttle (441) from the first position to the second position. Then, the carrier (462) is disengaged from the shuttle (441) in the second position and may retreat in a direction away from the sensor unit (100).

The carrier (462) includes a carrier body (463) to which the needle (485) is coupled, an elastically deformable carrier wing (471) extending from the carrier body (463), and a grip arm (479) connected to the side of the carrier body (463).

An insertion groove (465) is formed on one side of the carrier body (463). A portion of the needle (485) may be inserted into the insertion groove (465). A carrier clamp (467) may be provided in the insertion groove (465) to engage with the needle (485) and secure the needle (485). A fixing portion (469) to which the needle release driver (495) is coupled is provided on one side of the carrier body (463). Carrier (462) can relatively move with respect to shuttle (431) by needle release driver (495).

The needle release driver (495) provides a moving force to the carrier (462). The needle release driver (495) includes a needle release elastic member (496) whose one end is coupled to the fixing portion (457) of the shuttle (441) and the other end is coupled to the fixing portion (469) of the carrier (462). The needle release elastic member (496) is in the form of a tension spring and applies an elastic force to pull the carrier (462) toward the fixing portion (457) of the shuttle (441). That is, the needle release elastic member (496) can apply an elastic force to the carrier (462) in a direction away from the sensor unit (100) to move the needle (485) coupled to the carrier (462) to be separated from the sensor unit (100).

The needle release driver (495) can be changed to various other configurations that can provide a moving force to the carrier (462) in addition to the configuration including the needle release elastic member (496) in the form of a coil spring.

The carrier wing (471) is connected to the carrier body (463) in a shape that extends from the carrier body (463) in the moving direction of the carrier (462). The carrier wing (471) includes a wing body (472) elastically deformably connected to the carrier body (463), and a trigger (474) and a latch portion (477) protruding from the wing body (472).

The trigger (474) is provided on one side of the wing body (472) to protrude outward from the wing body (472). The trigger (474) is inserted into the slit (445) of the shuttle (441) and the slit (418) of the column portion (410) and can move along these slits (445 and 418 ). The trigger (474) is inserted into the slits (445 and 418) and guided by the slits (445 and 418), so that the carrier (462) can move linearly more stably. While the carrier (462) moves from the first position to the second position with the shuttle (441), the trigger (474) may contact the release portion (419) disposed in the middle of the slit (418) of the column member (410).

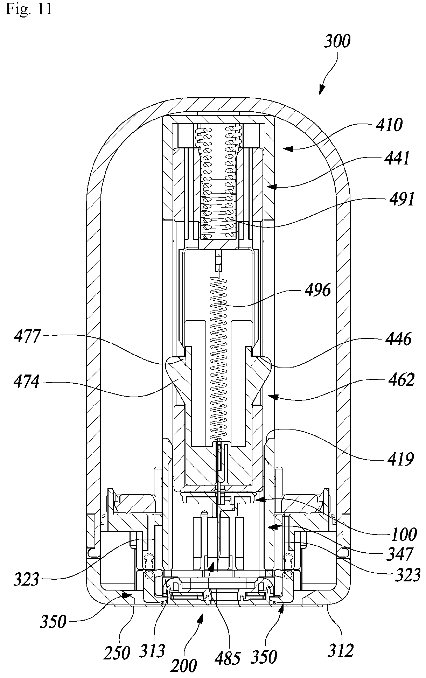

As shown in FIG. 11, the release portion (419) is placed in the middle of the slit (418) of the column member (410) to press the trigger (474). While the carrier (462) is moving from the first position to the second position, the trigger (474) may come into contact with the release portion (419) and the carrier wing (471) may be elastically deformed. The trigger (474) is provided with a trigger inclined portion (475) that contacts the release portion (420). While the carrier (462) is moving from the first position to the second position, the trigger inclined portion (475) is in contact with the release portion (419), so that the carrier wing (471) can be elastically deformed more smoothly, and the impact generated when the trigger (474) contacts the release portion (419) may be reduced.

The carrier latch (477) protrudes outward from the wing body (472). The direction in which the carrier latch (477) protrudes from the wing body (472) is the same as the direction in which the trigger (474) protrudes from the wing body (472). The protrusion height at which the carrier latch (477) protrudes from the wing body (472) is lower than the protrusion height at which the trigger (474) protrudes from the wing body (472). Carrier latch (477) may engage shuttle detent (446) of shuttle (441). In a state in which the carrier latch (477) is engaged with the shuttle detent (446), the carrier (462) can maintain the elastic member (496) in an elastically deformed state and cannot move in the direction in which the elastic force of the elastic member (496) acts. And, when the trigger (474) contacts the release portion (419) and the carrier wing (471) is elastically deformed, the carrier latch (477) may deviate from the shuttle detent (446) and the carrier (462) may move.

The grip arm (479) is provided on the side of the carrier body (463) to be elastically deformable. A pair of grip arms (479) are arranged to face each other on both sides of the carrier body (463) and engage with the sensor unit housing (120) of the sensor unit (100). The grip arm (479) is provided with a grip protrusion (480) engaged with the sensor unit housing (120) and a protrusion (481) in contact with the inner wall (436) of the column member (410). The carrier (462) is coupled to the shuttle (441) so that the carrier body (463) is located inside the shuttle (441) and the end of the grip arm (479) protrudes from the shuttle (441). The carrier (462) may fix the sensor unit (100) in such a way that a pair of grip arms (479) engage with the sensor unit housing (120). At this time, the surface of the sensor unit housing (120) is in close contact with the shuttle bottom portion (453) of the shuttle (431), and the pressing protrusion (455) of the shuttle (441) is inserted into the sensor unit groove (138) of the sensor unit housing (120). Therefore, the sensor unit (100) can remain stably coupled to the shuttle (441).

And the carrier (462) may position the sensor unit (100) in the first position when the insertion unit assembly (400) is coupled to the applicator body (300), as shown in FIG. 10. When the carrier (462) is located inside the column member (410), the protrusion (481) of the grip arm (479) comes in contact with the inner wall (436) of the column member (410). Accordingly, the grip arm (479) is biased in a direction in which it engages the sensor unit housing (120), and the carrier (462) can remain stably coupled to the sensor unit (100).

Meanwhile, when the sensor unit (100) and the carrier (462) move toward the base unit (200) and the sensor unit (100) is coupled to the base unit (200), the protrusion (481) is located in a space connected to the recess (313) in which the base unit (200) is accommodated and deviates from the inner wall (436) of the column member (410). Therefore, in a state in which the sensor unit (100) is coupled with the base unit (200), the fixing force of the grip arm (479) is weakened. As the sensor unit (100) is fixed to the base unit (200) by the adhesive layer (230), when the carrier (462) is pulled in a direction away from the body attachable unit (20) by the needle release driver (495), the grip arm (479) can be smoothly disengaged from the sensor unit (100) and separated from the sensor unit (100).

In addition to the configuration shown, the carrier (462) can be changed to various other configurations in which it is coupled to the needle (485) and installed to be relatively movable with respect to the shuttle (441). For example, the drawing shows a pair of carrier wings (471) being symmetrically provided on both sides of the carrier body (463), but the number or shape of the carrier wings (471) can be changed in various ways. Additionally, the number and shape of the grip arms (479) may be changed in various ways. Additionally, the coupling method of the carrier (462) and the needle (485) and the coupling method of the carrier (462) and the sensor unit (100) may also be changed in various ways.

The needle (485) is fixed to the carrier (462) and can move from the first position to the second position while coupled to the sensor unit (100). The needle (485) has a sharp tip so as to pierce the skin of a user and be smoothly inserted into the skin of a user. When the sensor unit (100) moves to the second position, the needle (485) penetrates the skin of a user before the sensor (110) and allows the sensor (110) to be stably inserted into the skin. The needle (485) is separated from the skin of a user after the sensor (110) is inserted into the skin of a user. The needle (485) includes a needle body (486) that can be inserted into the skin of a user, and a needle head (487) coupled to the carrier (462). The needle (485) can remain stably coupled to the carrier (462) by inserting the needle head (487) into the insertion groove (465) of the carrier (462) and engaging the carrier clamp (467).

The specific configuration of the needle (485) is not limited to that shown and may be changed in various ways. Additionally, the way the needle (485) is coupled to the carrier (462) may also vary.

The insertion unit assembly (400) may be coupled to the applicator body (300) in a state in which an elastic member (491) of the shuttle driver (490) for moving the shuttle (441) and an elastic member (496) of the needle release driver (495) for moving the needle assembly (460) are elastically deformed. As described above, in the process of coupling the insertion unit assembly (400) to the applicator body (300), since the shuttle (441) can be temporarily fixed to the column member (410) by the clamping portion (424), accidental movement of the shuttle (441) can be prevented. After the insertion unit assembly (400) is coupled to the applicator body (300), the clamping portion (424) is operated to release the restraining force on the shuttle (441) so that the insertion unit (440) is brought into an operable state by the operating member (365). The clamping portion (424) can be simply disengaged from the shuttle latch (448) of the shuttle (441). For example, when a user pulls the knob (427) to elastically deform the clamping arm (425), the clamping portion detent (426) may be released from the shuttle latch (448). At this time, the shuttle (441) is slightly pushed by the elastic member (491) of the shuttle driver (490), so that the clamping portion detent (426) is located in the guide groove (450) of the shuttle (441) and can be maintained out of the shuttle latch (448).

As such, the applicator assembly (10) according to an embodiment of the present disclosure can be simply assembled to the applicator body (300) by forming a single assembly in which the shuttle (441), a needle assembly (460), a shuttle driver (490), and a needle release driver (495), which constitute the insertion unit (440), together with the column member (410). Therefore, installation of the insertion unit (440) is easy, the task of installing the insertion unit (440) on the applicator body (300) can be partially automated, and manufacturing time can be reduced. Additionally, the sensor unit (100) can be simply installed on the applicator body (300) in a state coupled to the insertion unit (440) from the outside of the applicator body (300).

Additionally, the applicator assembly (10) according to an embodiment of the present disclosure may be coupled to the applicator body (300) in a locked state to prevent accidental operation of the insertion unit (440). And the insertion unit (440) can be converted to an operable state by simple manipulation after being coupled to the applicator body (300). Therefore, after installing the insertion unit (440) on the applicator body (300), there is no need for cumbersome follow-up work to convert the shuttle (441) and carrier (462) of the insertion unit (440) into an operable state.

As another exemplary embodiment, the insertion unit (440) is coupled to the applicator body (300) through the column member (410), and then the shuttle (441) and the carrier (462) can be moved to an operable position by a user.

The stopper member (500) is positioned to move linearly on the stage (331) of the middle frame (330). The stopper member (500) is installed to move in a direction intersecting the direction in which the sensor unit (100) moves from the first position to the second position. In the drawing, the stopper member (500) is shown to move in a direction perpendicular to the direction of movement of the sensor unit (100), but the movement direction of the stopper member (500) may be changed in various ways. The stopper member (500) may move from a third position, which supports the shuttle (441) to be positioned in the first position, to a fourth position where it disengages from the shuttle (441). The stopper member (500) may restrict the movement of the shuttle (441) placed in the first position, by being in contact with the shuttle protrusion (444) of the shuttle (441), which is located in the first position, at the third position, and when moving to the fourth position, the shuttle (441) may move away from the shuttle protrusion (444) to move to the second position.

The stopper member (500) includes a stopper member body (510) and a supporting portion (516) for supporting the shuttle (441).

The stopper member body (510) is provided with a stopper member opening (512) and a stopper member detent (513). The stopper member opening (512) is formed in the middle of the stopper member body (510) to penetrate the stopper member (500) in the thickness direction. The stopper member detent (513) is provided in a form that allows the hook portion (341) of the locking body (339) to engage with it. At one end of the stopper member body (510), a bracket portion (514) that can be contacted by the operating member (365) is provided. When the operating member (365) is operated by a user, the pressing protrusion (367) of the operating member (365) may contact the bracket portion (514) and push the stopper member (500).

Rail portions (515) are provided on both edges of the stopper member body (510). The rail portion (515) is formed to extend in a direction parallel to the moving direction of the stopper member (500) so that it can slide in contact with the guide protrusion (334) of the middle frame (330). Since the rail portion (515) is guided by the guide protrusion (334) of the middle frame (330), the stopper member (500) can stably move linearly without lifting on the stage (331) of the middle frame (330). As a method of guiding the stopper member (500) to move linearly, various other methods other than the method using the rail portion (515) and the guide protrusion (334) as shown may be used.

The supporting portion (516) is placed at the edge of the stopper member opening (512), The support portion (516) may protrude from the stopper member body (510) and support the shuttle protrusion (444) of the shuttle (441). When the stopper member (500) is positioned in the third position, the supporting portion (516) may support the shuttle protrusion (444) of the shuttle (441) positioned in the first position. And when the stopper member (500) moves to the fourth position, the supporting portion (516) deviates from the shuttle protrusion (444), so that the shuttle (441) can move to the second position. In addition to the shape that protrudes from the stopper member body (510), the supporting portion (516) can be changed into various other shapes that can contact or engage with the shuttle protrusion (444) or other parts of the shuttle (441).

The stopper member (500) cannot move when the locking body (339) is engaged with the stopper member detent (513). At this time, even if the operating member (365) is manipulated, the stopper member (500) cannot move from the third position and can maintain the state of supporting the shuttle (441). The stopper member (500) can operate the insertion unit (440) by moving the locking body (339) to the fourth position by the operating member (365) in a state in which the locking body (339) is disengaged from the stopper member detent (513).

The moving tab (600) is coupled to the applicator body (300) so that the protective sheet (240) provided on the base unit (200) can be removed by user manipulation. The moving tab (600) includes a moving tab body (610) coupled to the protective sheet (240), and a handle portion (621) extending from one side of the moving tab body (610). At least a portion of the moving tab (600) may be pulled out from the inside of the applicator body (300) to the outside through the entrance (328) of the applicator body (300). The moving tab (600) is supported on the stage (325) of the applicator body (300) and can move linearly in a direction perpendicular to the direction of movement of the sensor unit (100). The moving tab (600) may move from an initial position coupled with the protective sheet (240) of the base unit (200) located in the second position to a removal position where the protective sheet (240) is removed.

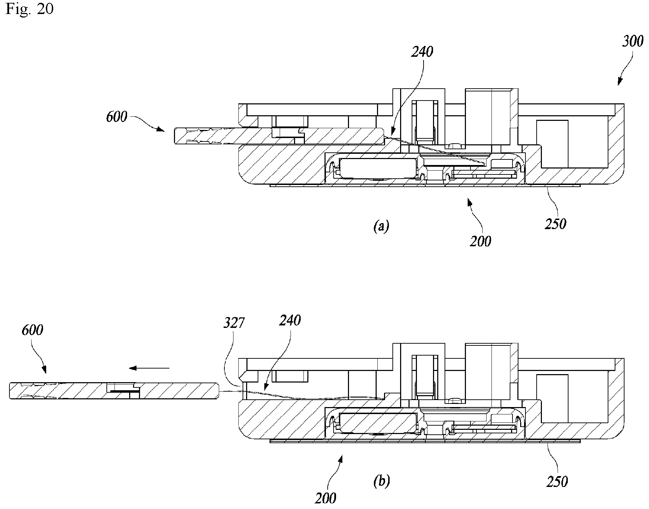

As shown in (a) of FIG. 20, when the moving tab (600) is located in the initial position, the moving tab body (610) is positioned in the applicator body (300) in a state coupled with the protective sheet (240), and the handle portion (621) protrudes from the applicator body (300). At this time, the adhesive layer (230) of the base unit (200) placed in the second position may remain covered by the protective sheet (240).

As shown in (b) of FIG. 20, when the moving tab (600) is pulled by a user and moves to the removal position, the moving tab (600) may remove the protective sheet (240) from the base unit (200) by pulling the protective sheet (240) in a direction away from the base unit housing (210).

The moving tab body (610) has a flat contact surface (611) to which a portion of the protective sheet (240) is coupled. The moving tab body (610) may be coupled to the protective sheet (240) by adhering the adhesive member (245) provided on the wing portion (242) of the protective sheet (240) to the contact surface (611). A grip portion (613) that can be inserted into the protective sheet hole (243) of the protective sheet (240) is provided in the middle of the contact surface (611). When the wing portion (242) of the protective sheet (240) is adhered to the contact surface (611), the grip portion (613) is inserted into the protective sheet hole (243), so that the moving tab body (610) can be more stably coupled to the protective sheet (240). The adhesive member (245) may be provided on the surface of the moving tab body (610). In this case, when the protective sheet (240) contacts the surface of the moving tab body (610), the adhesive member (245) may be adhered to the protective sheet (240).

Various other methods may be used to couple the moving tab (600) and the protective sheet (240). As another exemplary embodiment, the moving tab (600) may be coupled to the protective sheet (240) only by the adhesive member (245). Additionally, the moving tab (600) can be coupled to the protective sheet (240) only by inserting the grip portion (613) into the protective sheet hole (243). In addition, the moving tab (600) may be coupled to the protective sheet (240) in various ways, such as a method of engaging with the protective sheet (240).

Elastically deformable moving tab arms (615) are provided on both sides of the moving tab body (610). The moving tab arm (615) is provided with a moving tab latch (617) that can be engaged with the applicator body detent (326) of the applicator body (300). The moving tab latch (617) is provided with an inclined portion (618) that is inclined with respect to the moving direction of the moving tab (600). When the moving tab (600) is located in the initial position, the moving tab latch (617) engages the applicator body detent (326). Accordingly, the moving tab (600) is prevented from accidental movement and may move to the removal position only when a force greater than a preset amount is applied. When a force greater than a preset amount is applied to the moving tab (600), as the inclined portion (618) contacts the applicator body detent (326), the moving tab arm (615) can be smoothly elasticized and disengaged from the applicator body detent (326).

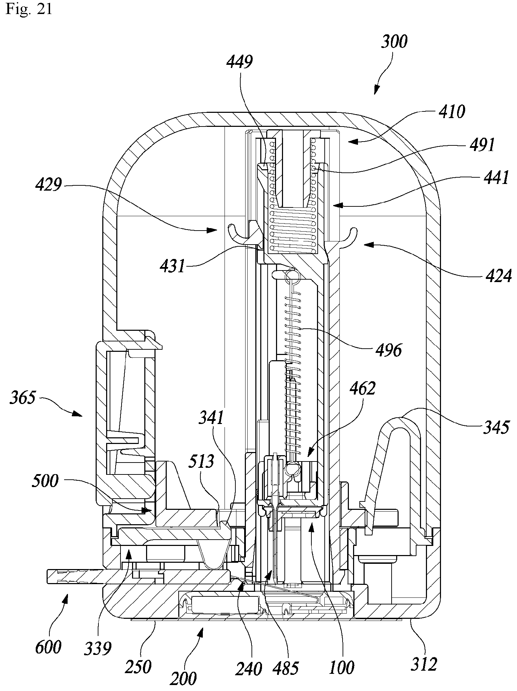

In addition to the function of removing the protective sheet, the moving tab (600) has a function of restricting the movement of the stopper member (500) together with the locking unit (338). As shown in FIG. 21, when the moving tab (600) is located in the initial position, the moving tab (600) supports the locking body (339) of the locking unit (338). At this time, as the leg portion (342) of the locking body (339) contacts to the moving tab (600), the locking body (339) is biased in a direction in which the hook portion (341) is engaged with the stopper member detent (513) of the stopper member (500) located at the third position. At this time, even if the operating member (365) is manipulated, the stopper member (500) cannot move.

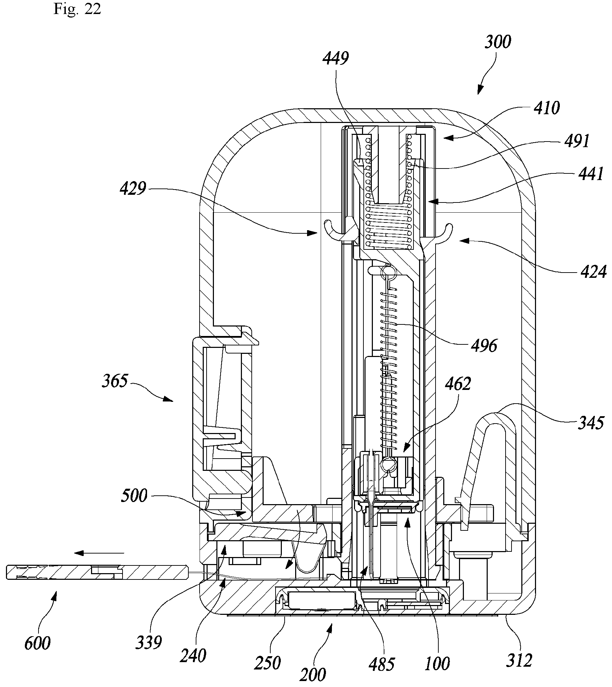

Meanwhile, as shown in FIG. 22, when the moving tab (600) is pulled by a user and moved to the removal position, the locking body (339) is released from the moving tab (600). At this time, the locking body (339) is returned to the original position so that the hook portion (341) is disengaged from the stopper member detent (513) of the stopper member (500). Therefore, the stopper member (500) is converted to a movable state by the operating member (365).

In this way, since the stopper member (500) can move after the moving tab (600) moves to the removal position, the insertion unit (440) can operate after the protective sheet (240) is removed from the base unit (200). Accordingly, the sensor unit (100) can be moved to the second position after the protective sheet (240) is removed from the base unit (200).

When the insertion unit (440) operates without the protective sheet (240) being completely separated and the sensor unit (100) moves to the second position, a problem in which the protective sheet (240) is caught between the sensor unit (100) and the base unit (200), or the sensor unit (100) is coupled with the base unit (200) without the protective sheet (240) being completely separated may occur. The applicator (30) according to this embodiment ensures that the separation operation of the protective sheet (240) is completed before the sensor unit (100) moves from the first position, so that it is possible to prevent the sensor unit (100) from reaching the base unit (200) without the protective sheet (240) being separated. Therefore, the risk of malfunction due to the carelessness of a user can be prevented.

The configuration of the moving tab (600) is not limited to that shown and can be changed in various ways. For example, in the drawing, the handle portion (621) is shown to protrude from the applicator body (300) when the moving tab (600) is located in the initial position, but the moving tab (600) may be installed on the applicator body (300) so that the handle portion (621) does not protrude from the applicator body (300) when positioned in the initial position. The drawing shows that the moving tab (600) completely exits the entrance (328) when moved to the removal position, but the moving tab (600) may be installed so that only a portion of the moving tab (600) exits through the entrance (328) and a portion of the moving tab (600) is located inside the applicator body (300) when moved to the removal position. As another exemplary embodiment, the moving tab may be installed as a push type. In this case, the moving tab is installed on the applicator body (300) to move linearly in a direction intersecting the moving direction of the sensor unit (100), and when a user pushes the moving tab, the protective sheet (240) can be removed or the binding force on the stopper member (500) or the operating member (365) can be released. As another exemplary embodiment, the moving tab may be installed to be movable in various directions on the applicator body (300) so as to be moved by a user.

Additionally, the drawing shows that the locking unit (338) is installed on the applicator body (300) so as to be moved by the moving tab (600), but the moving tab may have a configuration in which the locking unit is integrated as one piece. In this case, a locking body that can engage with the stopper member or the operating member may be provided on the moving tab in an elastically deformable form, or may be installed to be elastically supported. This moving tab may be disengaged from the stopper member or operating member when moved to the removal position.

Hereinafter, a process of attaching the body attachable unit (20) to the skin of a user using the applicator (30) according to an embodiment of the present disclosure will be described with reference to FIGS. 21 to 27.

As shown in FIG. 21, when the moving tab (600) is located in the initial position, the protective sheet (240) of the base unit (200) maintains the state of covering the adhesive layer (230). At this time, the locking body (339) of the locking unit (338) is in contact with the stopper member (500), and the movement of the stopper member (500) is restricted by the locking unit (338) and cannot move even if the operating member (365) is pressed.

As shown in FIG. 22, when the moving tab (600) is pulled by a user and moves to the removal position, the protective sheet (240) is removed from the base unit (200) by the moving tab (600). Then, the locking body (339) moves away from the moving tab (600) and is disengaged from the stopper member (500), and the applicator (30) is converted to an operable state.

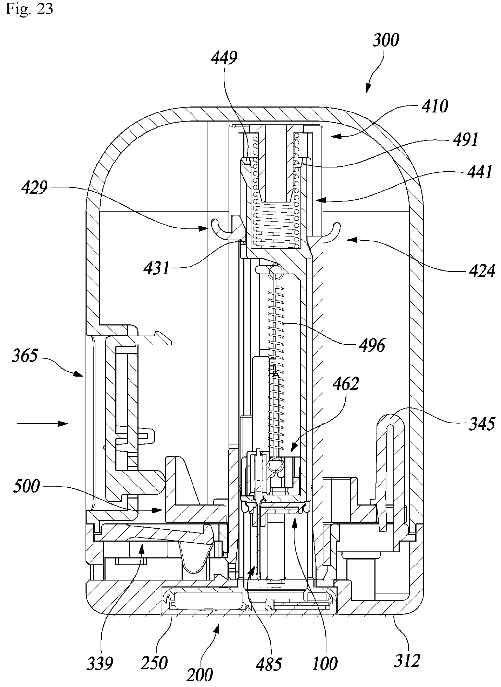

The applicator (30) can be operated with the bottom portion (312) of the applicator body (300) in contact with the skin of a user to move the sensor unit (100) toward the skin of a user. When the bottom portion (312) of the applicator body (300) comes into contact with the skin of a user, the base unit (200) is attached to the skin of a user through the adhesive portion (250). As shown in FIG. 23, when the operating member (365) is pressed by a user, the operating member (365) presses the stopper member (500). At this time, the stopper member (500) moves to the fourth position while compressing the return member (345).

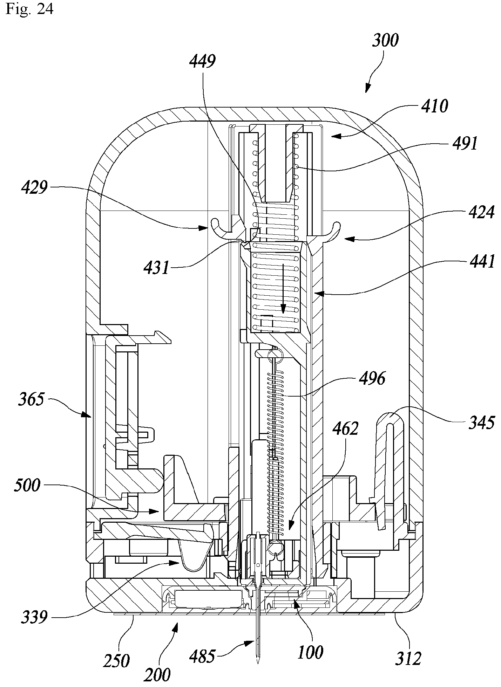

FIGS. 24 and 25, as the stopper member (500) moves from the third position to the fourth position, the shuttle (441) deviates from the stopper member (500) and moves to the second position by the elastic member (491). As the shuttle (441) moves to the second position, the needle (485) and the sensor (110) are inserted into the skin of a user, and by attaching the sensor unit (100) to the base unit (200) by the adhesive layer (230), the body attachable unit (20) is assembled. And, when the shuttle (441) reaches the second position, the shuttle (441) moves the locking hook (350) so that the locking hook (350) is disengaged from the base unit (200).

As previously described, the adhesive layer (230) couples the sensor unit (100) and the base unit (200), and seals between the sensor unit-electrical contact portion (146) of the sensor unit (100) and the base unit-electrical contact portion (225) of the base unit (200). AS the adhesive layer (230) seals the electrical connection portion (260) between the sensor unit (100) and the base unit (200), in a process in which the body attachable unit (20) is attached to the skin of a user and measures bio-signals, moisture, foreign substances, body fluids, etc. do not flow into the electrical connection portion (260) between the sensor unit (100) and the base unit (200). Therefore, the problem of malfunction of the body attachable unit (20) can be reduced, and stable operation of the body attachable unit (20) is possible.

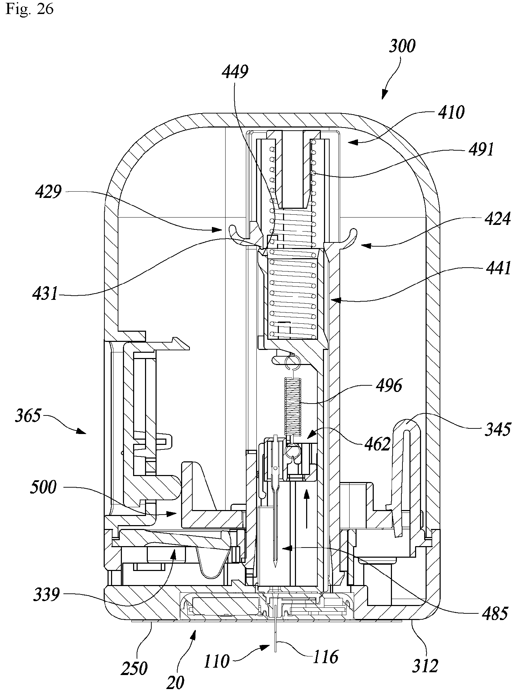

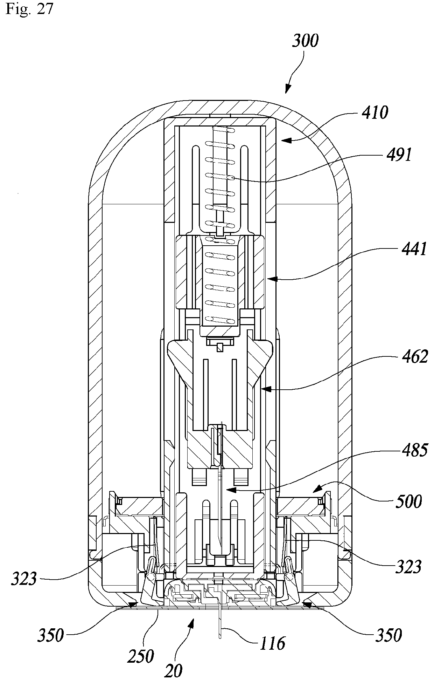

When the shuttle (441) moves to the second position and the insertion portion (116) of the sensor (110) and the needle (485) are inserted into the skin of a user, the trigger (474) of the carrier (462) comes into contact with release portion (419) of the column member (410). At this time, the carrier wing (471) of the carrier (462) is elastically deformed, thereby disengaging the carrier latch (477) from the shuttle detent (446). As shown in FIGS. 26 and 27, the carrier (462) is disengaged from the shuttle (441) and moves in a direction away from the skin of a user by the needle release driver (495). At this time, the needle (485) comes out of the skin of a user and the body attachable unit (20) remains attached to the skin of a user by the adhesive portion (250).

As described above, after the insertion portion (116) of the sensor (110) is inserted into the skin, in the process of the needle (485) coming out of the skin of a user, the retention protrusion (136) of the sensor unit housing (120) contacts the middle portion (113) of the sensor (110) to prevent the insertion portion (116) from moving. Therefore, the insertion portion (116) cannot retract in the same direction as the needle (485) and can remain stably inserted into the skin.

Additionally, before the applicator (30) is separated from the body attachable unit 20, the shuttle bottom portion (453) of the shuttle (441) located in the second position maintains a state being in contact with the sensor unit (100). And the pressing protrusion (455) of the shuttle (441) remains inserted into the sensor unit groove (138) of the sensor unit (100). Therefore, when the needle (485) comes out of the skin of a user, the shuttle (441) can support the sensor unit (110) without moving, and the insertion portion (116) of the sensor (110) can remain stably inserted into the skin.

After the body attachable unit (20) is attached to the skin of a user, the applicator (30) is separated from the body attachable unit (20). Additionally, the body attachable unit (20) can measure the biometric information of a user and transmit the measurement information to the external terminal (5) or the like.

As described above, since the applicator assembly (10) according to an embodiment of the present disclosure is mounted on the applicator (30) with the adhesive layer (230) of the base unit (200) covered with the protective sheet (240), even if it is not used for a long time, the adhesiveness of the adhesive layer (230) does not deteriorate.

Additionally, in the applicator assembly (10) according to an embodiment of the present disclosure, after a user removes the protective sheet (240) of the base unit (200) by manipulating the moving tab (600), the insertion unit (440) operates to move the sensor unit (100) toward the base unit (200). Therefore, malfunctions by carelessness of a user can be prevented and safe use is possible.

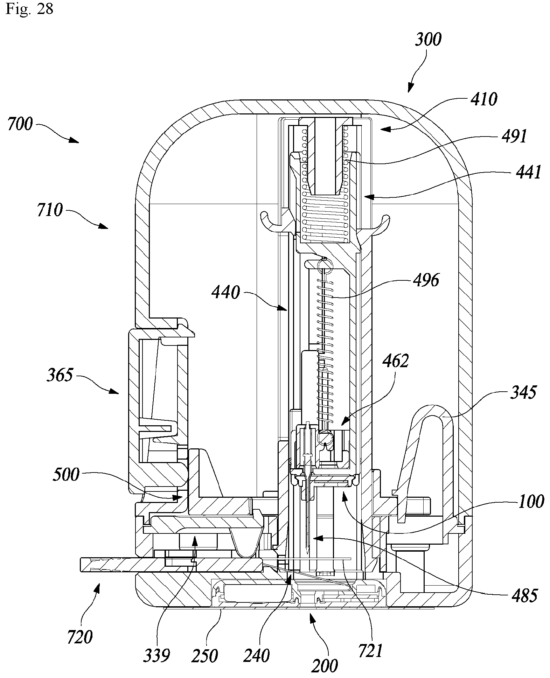

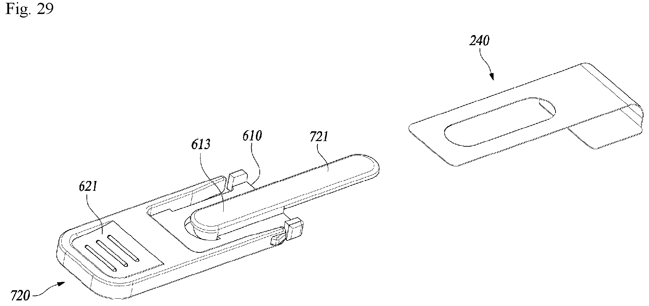

Meanwhile, FIG. 28 is a cross-sectional view showing an applicator assembly according to another embodiment of the present disclosure, and FIG. 29 shows a protective sheet and a moving tab of the applicator assembly shown in FIG. 28.

The applicator assembly (700) shown in FIG. 28 includes a sensor unit (100), a base unit (200) that is attached to the skin of a user and coupled to the sensor unit (100), and an applicator (710) that couples the sensor unit (100) and the base unit (200) and inserts the sensor (110) of the sensor unit (100) into the skin of a user. The applicator (710) includes an applicator body (300) to which the base unit (200) is detachably coupled, an operating member (365) installed on the applicator body (300) for user manipulation, a column member (410) fixed to the applicator body (300), an insertion unit (440) coupled to the applicator body (300) through a column member (410) so as to move the sensor unit (100) toward the base unit (200), a stopper member (500) movably installed on the applicator body (300), and a moving tab (720) for separating the protective sheet (240) of the base unit (200).

The moving tab (720) is coupled to the applicator body (300) so that the protective sheet (240) provided on the base unit (200) can be removed by being manipulated by a user. The moving tab (720) can be operated by a user to remove the protective sheet (240) by moving from the initial position to the removal position. The moving tab (600) includes a moving tab body (610) coupled to the protective sheet (240), and a handle portion (621) extending from one side of the moving tab body (610). The moving tab body (610) is provided with a grip portion (613) to which the protective sheet (240) is coupled. Additionally, the moving tab body (610) is provided with a cover portion (721). The cover portion (721) is provided in a form that can cover the upper part of the protective sheet (240) coupled to the grip portion (613). When the moving tab (720) is located in the initial position, the cover portion (721) is located between the needle (485) coupled to the sensor unit (100) and the protective sheet (240). The cover portion (721) may prevent the needle (485) from coming into contact with the protective sheet (240) in a case in which the protective sheet (240) is removed from the base unit (200) or while the protective sheet (240) is removed from the base unit (200). The shape or location of the cover portion (721) may be changed in various ways.

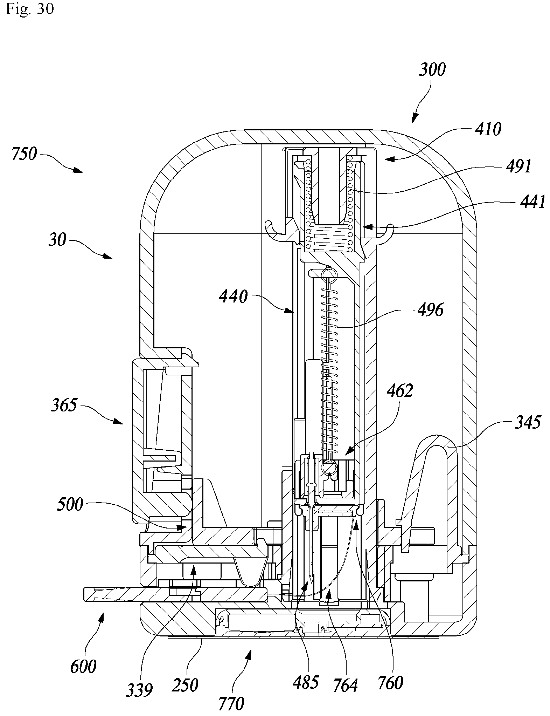

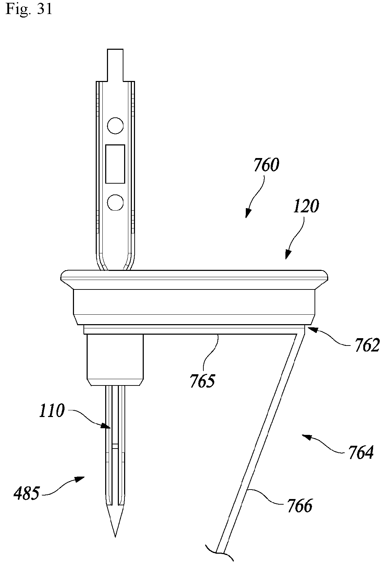

Meanwhile, FIG. 30 is a cross-sectional view showing an applicator assembly according to another embodiment of the present disclosure, and FIG. 31 shows a sensor unit of the applicator assembly shown in FIG. 30.

The applicator assembly (750) shown in FIG. 30 includes a sensor unit (760), a base unit (770) that is attached to the skin of a user and coupled to the sensor unit (760) and an applicator (30) that couples the sensor unit (760) and the base unit (770) and inserts the sensor of the sensor unit (760) into the skin of a user. The applicator (30) includes an applicator body (300) to which the base unit (770) is detachably coupled, an operating member (365) installed on the applicator body (300) for user operation, a column member (410) fixed to the applicator body (300), an insertion unit (440) coupled to the applicator body (300) through a column member (410) so as to move the sensor unit (760) toward the base unit (770), a stopper member (500) movably installed on the applicator body (300), and a moving tab (600) for separating the protective sheet (764) of the sensor unit (760).

The sensor unit (760) includes a sensor (110) inserted into the skin of a user, a sensor unit housing (120) to which the sensor (110) is coupled, an adhesive layer (762) provided on the sensor unit housing (120) to be attached to the base unit (770), and a protective sheet (764) covering the adhesive layer (762). The protective sheet (764) includes a protective portion (765) adhered to the adhesive layer (762) and a wing portion (766) extending from an edge of the protective portion (765). The wing portion (766) may extend a certain length from the edge of the sensor unit housing (120) and be coupled to the moving tab (600). The protective sheet (764) may be coupled to the moving tab (600) in the same manner as previously described.

The sensor unit (760) is mounted on the applicator (30) in a state in which the protective sheet (764) is attached to the adhesive layer (762). The protective sheet (764) may be separated from the adhesive layer (762) by the moving tab (600) before the sensor unit (760) moves from the first position to the second position. After the protective sheet (764) is removed from the sensor unit (760) by the moving tab (600), the sensor unit (100) may be moved toward the base unit (770) by the insertion unit (440) and coupled to the base unit (770).

The applicator (30) of this embodiment may include a moving tab (720) provided with a cover portion (721), as shown in FIG. 29. this moving tab (720) can prevent a case in which the protective sheet (764) is maintained to cover the adhesive layer (762) of the sensor unit (760) or prevent the needle (485) from coming into contact with the protective sheet (764) while the protective sheet (764) is removed from the sensor unit (760).

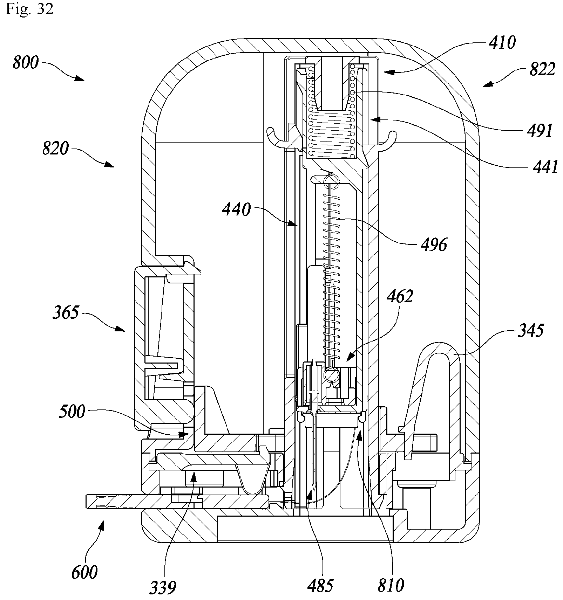

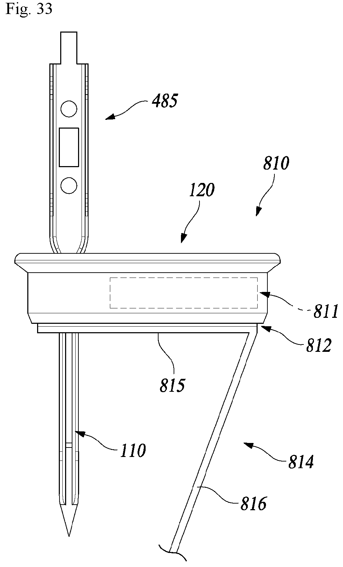

Meanwhile, FIG. 32 is a cross-sectional view showing an applicator assembly according to another embodiment of the present disclosure, and FIG. 33 shows a sensor unit of the applicator assembly shown in FIG. 32.

The applicator assembly (800) shown in FIG. 32 includes a sensor unit (810) and an applicator (820) that inserts the sensor of the sensor unit (810) into the skin of a user. The applicator (820) includes an applicator body (822), an operating member (365) installed on the applicator body (300) for user operation, a column member (410) fixed to the applicator body (300), an insertion unit (440) coupled to the applicator body (822) through a column member (410) to move the sensor unit (810) from a first position to a second position, a stopper member (500) movably installed on the applicator body (822), amd a moving tab (600) for separating the protective sheet (814) of the sensor unit (810).

The sensor unit (810) includes a sensor (110) inserted into the skin of a user, a sensor unit housing (120) to which the sensor (110) is coupled, an electronic component (811) disposed inside the sensor unit housing (120), an adhesive layer (812) provided on the sensor unit housing (120) to be attached to the skin of a user, and a protective sheet (814) covering the adhesive layer (812). The electronic component (811) includes a circuit board electrically connected to the sensor (110), a process chip installed on the circuit board to convert biometric information measured by the sensor (110) into an electrical signal, a communication chip for communication with the outside, a battery, etc. The sensor unit (810) may be attached to the skin of a user by moving from the first position to the second position in a state being coupled with the needle (485). The protective sheet (814) includes a protective portion (815) adhered to the adhesive layer (812) and a wing portion (816) extending from an edge of the protective portion (815). The wing portion (816) may extend by a certain length from the edge of the sensor unit housing (120) and be coupled to the moving tab (600). The protective sheet (814) may be coupled to the moving tab (600) in the same manner as described above.

The sensor unit (810) is mounted on the applicator (820) in a state in which the protective sheet (814) is attached to the adhesive layer (812). The protective sheet (814) may be separated from the adhesive layer (812) by the moving tab (600) before the sensor unit (810) moves from the first position to the second position. After the protective sheet (814) is separated from the sensor unit (810) by the moving tab (600), the sensor unit (810) may be moved from the first position to the second position by the insertion unit (440). The sensor unit (810) may be inserted into the skin of a user and attached to the skin of a user by an adhesive layer (812) in the second position. After the sensor unit (810) is attached to the skin of a user, the needle (485) is separated from the skin, and the sensor unit (810) is separated from the applicator (820) to measure the biometric information of a user.

The applicator assembly (800) according to this embodiment can be operated without the need for a separate base unit because the sensor unit (810) can be directly attached to the skin of a user and has signal processing function and communication function. That is, the sensor unit (810) can be attached to the skin of a user, measure biometric information, and transmit the measured biometric information to an external terminal (5), etc.

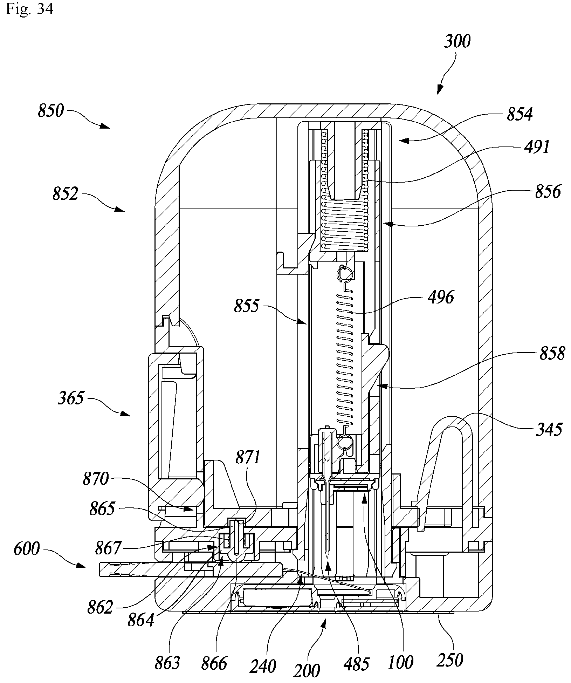

Meanwhile, FIG. 34 is a cross-sectional view showing an applicator assembly according to another embodiment of the present disclosure.

The applicator assembly (850) shown in FIG. 34 includes a sensor unit (100), a base unit (200) that is attached to the skin of a user and coupled to the sensor unit (100), and an applicator (852) that couple the sensor unit (100) and the base unit (200) and inserts the sensor (110) of the sensor unit (100) into the skin of a user. The applicator (852) includes an applicator body (300) to which the base unit (200) is detachably coupled, an operating member (365) installed on the applicator body (300) for user operation, and a column member (854) fixed to the applicator body (300), an insertion unit (855) coupled to the applicator body (300) through a column member (854) so as to move the sensor unit (100) toward the base unit (200), a stopper member (870) movably installed on the applicator body (300), and a moving tab (600) for separating the protective sheet (240) of the base unit (200). The insertion unit (855) includes a shuttle (856) movably installed inside the column member (854), a carrier (858) and a needle (485) that can move together with the shuttle (856).

A locking unit (862) is installed on the applicator body (300) to restrict the movement of the stopper member (870). The locking unit (862) includes a locking body (863) that can move in a direction intersecting the moving direction of the moving tab (600) so as to be engaged with the stopper member (870) or disengaged from the stopper member (870), and an elastic member (867) that applies elastic force to the locking body (863). The locking body (863) includes a body portion (864), a hook portion (865) connected to the body portion (864) to engage with the stopper member (870), and a leg portion (866) connected to the body portion (864) so as to contact the moving tab (600). The elastic member (867) has one end in contact with the applicator body (300) and the other end in the form of a coil spring in contact with the body portion (864). The elastic member (867) applies elastic force in the direction in which the locking body (863) is disengaged from the stopper member detent (871) of the stopper member (870). The locking body (863) is biased in a direction in which the hook portion (865) is engaged with the stopper member detent (871) of the stopper member (870) while compressing the elastic member (867) when the leg portion (866) is in contact with the moving tab (600).

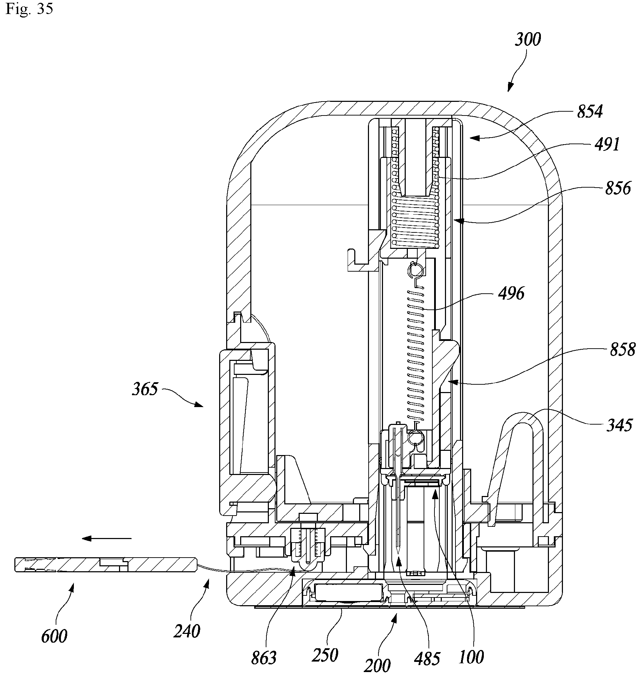

As shown in FIG. 35, when the moving tab (600) moves to separate the protective sheet (240) by a user, the leg portion (866) of the locking body (863) deviates from the moving tab (600). At this time, the locking body (863) is biased by the elastic member (867) in the direction in which the hook portion (865) is disengaged from the stopper member detent (871), and the stopper member (870) is converted to a movable state by the operating member (365).

In this embodiment, the shape of the locking body (863) can be changed in various ways. Additionally, the drawing shows that the locking body (863) can move in a direction perpendicular to the moving direction of the moving tab (600), but the locking body (863) can be installed to move in another direction. Additionally, the elastic member (867) may be changed to another form that can apply elastic force to the locking body (863) in addition to the coil spring form, and the installation location of the elastic member (867) may also be changed.

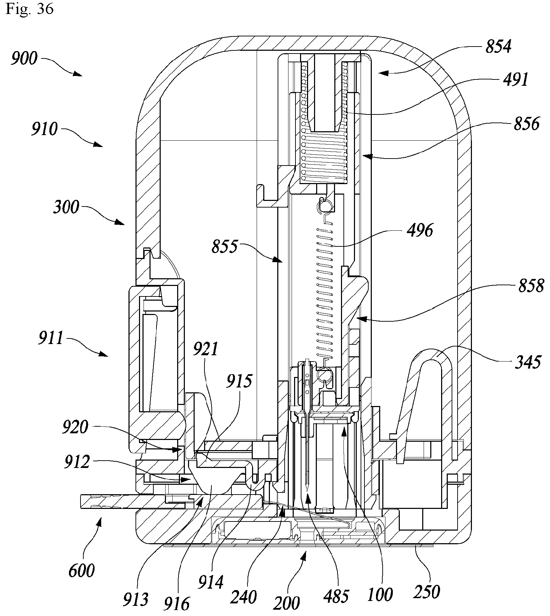

Meanwhile, FIG. 36 is a cross-sectional view showing an applicator assembly according to another embodiment of the present disclosure.

The applicator assembly (900) shown in FIG. 36 includes a sensor unit (100), a base unit (200) that is attached to the skin of a user and coupled to the sensor unit (100), and an applicator (910) that couples the sensor unit (100) and the base unit (200) and inserts the sensor (110) of the sensor unit (100) into the skin of a user. The applicator (910) includes an applicator body (300) to which the base unit (200) is detachably coupled, an operating member (911) installed on the applicator body (300) for user manipulation, a column member (854) fixed to the applicator body (300), an insertion unit (855) coupled to the applicator body (300) through a column member (854) so as to move the sensor unit (100) toward the base unit (200), a stopper member (920) movably installed on the applicator body (300), and a moving tab (600) for separating the protective sheet (240) of the base unit (200).

A locking unit (912) is installed on the applicator body (300) to restrict the movement of the stopper member (920). The locking unit (912) includes a locking body (913) that is elastically deformably coupled to the applicator body (300) so as to be engaged with or disengaged from the stopper member (920). The locking body (913) includes a body portion (914), a hook portion (915) connected to the body portion (914) so as to engage with the stopper member detent (921) of the stopper member (920), and a leg portion (916) connected to the body portion (914) so as to come into contact with the moving tab (600).

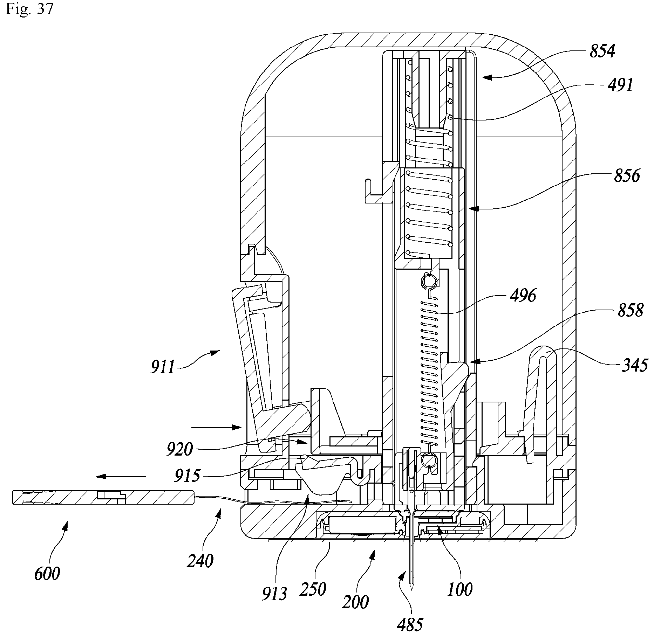

As shown in FIG. 37, when the moving tab (600) moves to separate the protective sheet (240) by the user, the leg portion (916) of the locking body (913) deviates from the moving tab (600). At this time, the locking body (913) is biased in the direction in which the hook portion (915) is disengaged from the stopper member detent (921), and the stopper member (920) is converted to a movable state by the operating member (365). When the operating member (365) is operated by a user, the stopper member (920) moves from the third position to the fourth position, and as the insertion unit (440) operates, the sensor (110) of the sensor unit (100) can be inserted into the skin of a user.

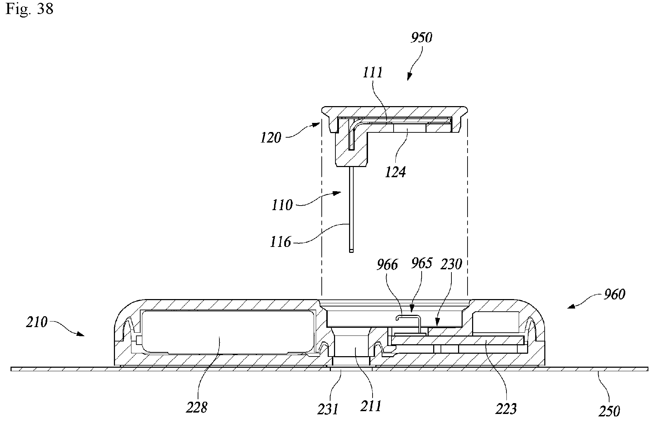

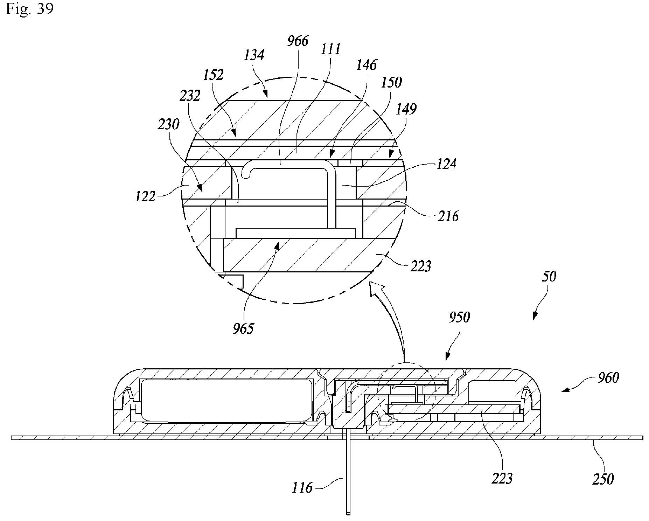

Meanwhile, FIGS. 38 and 39 show other embodiments of the sensor unit and the base unit.

The sensor unit (950) shown in FIGS. 38 and 39 includes a sensor (110) inserted into the skin of a user, a sensor unit housing (120) to which the sensor (110) is coupled, and a sensor adhesive portion (149) and an adhesive pad (152) for fixing the sensor (110) to the sensor unit housing (120). The sensor unit (950) has a configuration in which the sensor unit-electrical contact portion for electrically connecting the sensor (110) to the base unit (960) is omitted.

The base unit (960) includes a base unit housing (210) to which the sensor unit (950) is coupled, a circuit board (223) installed inside the base unit housing (210), a battery (228), an adhesive layer (230) provided on the base unit housing (210) to be attached to the sensor unit (950), and a base unit-electrical contact portion (965) that is electrically connected to the sensor (110) of the sensor unit (950). The adhesive layer (230) may be covered and protected with a protective sheet (not shown) and then removed before the sensor unit (950) is coupled to the base unit (960). An adhesive portion (250) is provided on the surface of the base unit housing (210). The base unit-electrical contact portion (965) contact sensor (110) when sensor unit (950) is coupled to base unit (960). The base unit-electrical contact portion (965) is electrically connected to the circuit board (223), and a portion of if may protrude from the supporting surface (216) and contact the sensor (110). The base unit-electrical contact portion (965) may include a terminal portion (966) that contacts an electrode provided on the sensor body (111) of the sensor (110). The terminal portion (966) may electrically connect the sensor (110) and the circuit (223) by contacting the electrode of the sensor body (111). The terminal portion (966) may be formed to be elastically deformed when in contact with the sensor body (111) so as to stably contact the sensor body (111).

The sensor unit (950) constitutes the body attachable unit (50) along with the base unit (960) by being attached to the base unit (960) by the adhesive layer (230). The sensor unit (950) is attached to the base unit (960) by the adhesive layer (230), thereby forming a body attachable unit (50) with the base unit (960). When the sensor unit (950) is coupled to the base unit (960), the base unit-electrical contact portion (965) may pass through the through hole (124) and the sensor adhesive opening (150), and then contact the sensor body (111). Accordingly, the sensor (110) may be electrically connected to the base unit (960) by the base unit-electrical contact portion (965). At this time, the adhesive layer (230) seals between the sensor (110) and the base unit-electrical contact portion (965) to prevent moisture or foreign substances from entering the electrical connection portion between the sensor unit (950) and the base unit (960).

In order to electrically connect the sensor (110) and the base unit (960), the specific configuration, number, and location of the base unit-electrical contact portion provided in the base unit (960) may be changed in various ways.

Although the present disclosure has been described above with preferred examples, the scope of the present disclosure is not limited to the form described and shown above.

For example, the drawing shows that the sensor unit is detachably coupled to a carrier which is coupled with a needle and moves from a first position to a second position, but the sensor unit is detachably coupled to the shuttle or a separate member which is provided in the shuttle and can move with the shuttle.

In addition, the drawing shows that the stopper member is moved from the third position to the fourth position by the operating member operated by a user, but the applicator may include a driver which is capable of providing a moving force to the stopper member. In this case, the operating member which is operated by a user to operate the driver may allow the stopper member to move from the third position to the fourth position by the driver.

Additionally, the drawing shows that the moving tab for the removal of the protective sheet interacts with the locking unit, but the moving tab may be installed so as not to affect the movement of the locking unit. In this case, the applicator may comprise a separate component that can interact with the locking unit. If necessary, the locking unit may be omitted.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

The invention may further include one or more of the following items:
[Item 1]
   An applicator for inserting a sensor for measuring biometric information into skin of a user, the applicator comprising: an applicator body; an insertion unit installed to the applicator body to move a sensor unit, which includes the sensor, a sensor unit housing to which the sensor is mounted, an adhesive layer provided at the sensor unit housing, and a protective sheet covering an adhesive layer, from a first position spaced apart from the skin of the user to a second position where the sensor is inserted into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.
[Item 2]
   The applicator according to Item 1, wherein the moving tab is installed to the applicator body to be movable in a direction of intersecting a movable direction of the sensor unit.
[Item 3]
   The applicator according to Item 1, wherein the moving tab is installed to the applicator body to move in a direction perpendicular to a movable direction of the sensor unit.
[Item 4]
   The applicator according to Item 1, wherein: an opening is provided at one side of the applicator body, and the moving tab is configured to move in a direction of intersecting a movable direction of the sensor unit such that at least a portion of the moving tab is drawn out from an inside of the applicator body to an outside of the applicator body through the opening.
[Item 5]
   The applicator according to Item 4, wherein the moving tab comprises: a moving tab body located inside the applicator body and coupled with the protective sheet, and a handle portion extending from one side of the moving tab body and exposed to the outside of the applicator body for the user to be able to hold the handle portion with a hand.
[Item 6]
   The applicator according to Item 5, wherein a moving tab arm configured to be engageable with an applicator body detent provided at the applicator body is provided to be elastically deformable at one side of the moving tab body, and the moving tab arm is configured to, when a force greater than a preset amount is applied to the moving tab, be disengaged from the applicator body detent to be withdrawn from the opening.
[Item 7]
   The applicator according to Item 1, wherein the moving tab is coupled to the protective sheet by an adhesive member.
[Item 8]
   The applicator according to Item 1, wherein the protective sheet comprises: a protective portion covering the adhesive layer, a wing portion extended from an edge of the protective portion, and a protective sheet hole formed at one side of the wing portion, and wherein the moving tab has a grip portion corresponding to the protective sheet hole, and is coupled with the protective sheet in a way that the grip portion is inserted into the protective sheet hole.
[Item 9]
   The applicator according to Item 1, comprising: an operation member installed to the applicator body to be operated by the user; and a stopper member movably installed to the applicator body to operate the insertion unit in association with the operation member, and wherein the moving tab is configured to restrain movement of the stopper member in an initial position where the protective sheet is coupled with the adhesive layer to cover the adhesive layer, and release restraint on the stopper member in a removal position where the protective sheet is separated from the adhesive layer.
[Item 10]
   The applicator according to Item 9, comprising a locking unit including a locking body installed to the applicator body to be engaged with the stopper member or disengaged from the stopper member, and wherein the moving tab is configured to bias the locking body to be engaged with the stopper member by contacting the locking body in the initial position, and wherein the locking body is configured to, when the moving tab moves to the removal position, be disengaged from the stopper member.
[Item 11]
   The applicator according to Item 10, wherein the locking body composing: a body portion connected to the applicator body, wherein an angle of the body portion is changeable, a hook portion connected to one side of the body portion to be engaged with the stopper member, and a leg portion connected to another side of the body portion to contact the moving tab, and wherein the locking body is configured to, when the moving tab is located in the initial position, be biased in a direction in which the hook portion is engaged with the stopper member by contact of the leg portion with the moving tab, and the leg portion is configured to, when the moving tab moves to the removal position, move away from the moving tab and be biased in a direction in which the hook portion is disengaged from the stopper member.
[Item 12]
   The applicator according to Item 11, wherein: the applicator body includes a stage which movably supports the stopper member, and the body portion is arranged in a through hole formed at one side of the stage to penetrate the stage.
[Item 13]
   The applicator according to Item 10, wherein the locking unit comprises an elastic member configured to apply elastic force to the locking body in a direction of being away from the stopper member, and wherein the locking body is configured to, when the moving tab is located in the initial position, contact the moving tab to be biased in a direction of being engaged with the stopper member, and, when the moving tab moves to the removal position, deviate from the moving tab to be biased in a direction of being released from the stopper member by the elastic force of the elastic member.
[Item 14]
   The applicator according to Item 1, wherein the insertion unit includes a needle which is movable from the first position to the second position to be inserted into the skin of the user together with the sensor, and wherein the moving tab includes a cover portion arranged between the protective sheet and the needle to prevent the needle from contacting the protective sheet.
[Item 15]
   An applicator for inserting a sensor into skin of a user, the applicator comprising: an applicator body to which a base unit including a base unit housing, and an adhesive portion provided at the base unit housing to be attached to the skin of the user is separably coupled; an insertion unit installed to the applicator body to move the sensor unit, which includes the sensor and a sensor unit housing to which the sensor is mounted, an adhesive layer provided at the sensor unit housing to be attachable to the base unit housing, and a protective sheet covering the adhesive layer from a first position which is spaced apart from the base unit to a second position where the sensor unit is coupled with the base unit to insert the sensor into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.
[Item 16]
   An applicator for inserting a sensor into skin of a user, the applicator comprising: an applicator body to which a base unit including a base unit housing, an adhesive layer provided at one side of the base unit housing, a protective sheet covering the adhesive layer, and an adhesive portion provided at another side of the base unit housing to be attached to the skin of the user is separably coupled; an insertion unit installed to the applicator body to move the sensor unit, which includes the sensor and a sensor unit housing which is configured to be attachable to the base unit housing by the adhesive layer and the sensor is mounted to, from a first position which is spaced apart from the base unit to a second position where the sensor unit is coupled with the base unit to insert the sensor into the skin of the user; and a moving tab to which a portion of the protective sheet is coupled and is movably installed to the applicator body to be movable by the user to move the protective sheet in a direction of being away from the base unit housing to separate the protective sheet from the adhesive layer.
[Item 17]
   An applicator assembly comprising: an applicator body; a sensor unit including a sensor, a sensor unit housing to which the sensor is mounted, an adhesive layer provided at the sensor unit housing, and a protective sheet covering the adhesive layer; an insertion unit installed to the applicator body to move the sensor unit from a first position spaced away from the skin of the user to a second position where the sensor is inserted into the skin of the user; and a moving tab to which a portion of the protective sheet is coupled and is movably installed to the applicator body to be movable by the user to move the protective sheet in a direction of being away from the sensor unit housing to separate the protective sheet from the adhesive layer.
[Item 18]
   The applicator assembly according to Item 17, comprising a base unit including a base unit housing to which the sensor unit housing is coupled and an adhesive portion provided at the base unit housing to be attached to the skin of the user, and spaced apart from the sensor unit and separably coupled to the applicator body, and wherein the sensor unit housing is configured to be attachable to the base unit housing by the adhesive layer in the second position.
[Item 19]
   An applicator assembly comprising: a sensor unit including a sensor and a sensor unit housing to which the sensor is mounted; a base unit including a base unit housing, an adhesive layer provided at one side of the base unit housing to attach the sensor unit housing and the base unit housing, a protective sheet covering the adhesive layer, and an adhesive portion provided at another side of the base unit housing to be attached to the skin of the user; an applicator body to which the base unit is separably coupled; an insertion unit mounted to the applicator body to insert the sensor into the skin of the user by moving the sensor unit from a first position spaced apart from the base unit to a second position where the sensor unit coupled with the base unit; and a moving tab to which a portion of the protective sheet is coupled and is movably installed to the applicator body to be movable by the user to move the protective sheet in a direction of being away from the base unit housing to separate the protective sheet from the adhesive layer.

## Claims

1. An applicator assembly (10) comprising:
an applicator body (300);
a base unit (200, 960) separably coupled to the applicator body (300), and including a base unit housing (210) and a base unit electrical contact portion (225, 965);
a sensor unit (100, 950) including a sensor (110) and a sensor unit housing (120) to which the sensor (110) is mounted;
an insertion unit (440) installed to the applicator body (300) to move the sensor unit (100, 950) from a first position spaced apart from the base unit (200, 960) to a second position where the sensor unit (100, 950) is coupled with the base unit (200, 960); and
an adhesive layer (230) provided between the sensor unit housing (120) and the base unit housing (210) to attach the sensor unit housing (120) to the base unit housing (210) in the second position,
wherein the adhesive layer (230) includes an adhesive layer opening (232),
wherein the base unit electrical contact portion (225, 965) is electrically connected to the sensor unit (100, 950) in the second position through the adhesive layer opening (232), and
wherein the adhesive layer (230) is configured to seal a periphery of the adhesive layer opening (232).

2. The applicator assembly (10) according to claim 1,
wherein the sensor unit (100) further includes a sensor unit electrical contact portion (146); and
wherein the base unit electrical contact portion (225) contacts the sensor unit electrical contact portion (146) in the second position through the adhesive layer opening (232).

3. The applicator assembly (10) according to claim 1,
wherein the base unit electrical contact portion (965) is configured to directly contact the sensor (110) in the second position through the adhesive layer opening (232).

4. The applicator assembly (10) according to any one of claims 1 to 3,
wherein the base unit housing (210) includes a mounting portion (212) having a base unit recess (213) and a supporting surface (216) provided inside the base unit recess (213),
wherein the sensor unit housing (120) includes a housing body (142) configured to be fitted into the base unit recess (213), and
wherein the adhesive layer (230) is disposed on the supporting surface (216).

5. The applicator assembly (10) according to claim 4,
wherein the base unit recess (213) includes a first recess (214) and a second recess (215) that is wider than the first recess (214), and
wherein the housing body (142) includes a body portion (143) corresponding to the first recess (214) and a cover portion (144) corresponding to the second recess (215), such that the sensor unit housing (120) is stably fitted into the base unit housing (210).

6. The applicator assembly (10) according to claim 4 or 5, wherein the base unit electrical contact portion (225, 965) is arranged on the mounting portion (212); and
wherein the base unit electrical contact portion (225, 965) is at least partially exposed to an outside of the adhesive layer (230) through the adhesive layer opening (232).

7. The applicator assembly (10) according to any one of claims 1 to 6, further comprising:
a protective sheet (240) covering and protecting the adhesive layer (230) in the first position,
wherein the protective sheet (240) is configured to be separated from the adhesive layer (230) before the sensor unit (100, 950) is moved to the second position.

8. The applicator assembly (10) according to claim 7, further comprising:
a moving tab (600) to which a portion of the protective sheet (240) is coupled,
wherein the moving tab (600) is movably installed to the applicator body (300), and
wherein the protective sheet (240) is separated from the adhesive layer (230) by moving the moving tab (600) in a direction away from the base unit housing (210).

9. The applicator assembly (10) according to any one of claims 1 to 8,
wherein the sensor unit (100, 950) further includes a sensor adhesive portion (149) disposed between a housing base (121) of the sensor unit housing (120) and the sensor (110) to attach the sensor (110) to the housing base (121),
wherein the sensor adhesive portion (149) includes a sensor adhesive portion opening (150) through which the sensor (110) is in contact with the base unit electrical contact portion (965) or the sensor unit electrical contact portion (146), and
wherein the sensor adhesive portion (149) is configured to seal a periphery of the sensor adhesive portion opening (150).

10. The applicator assembly (10) according to any one of claims 1 to 9, wherein the adhesive layer (230) is provided on the base unit housing (210) to be attached to the sensor unit (100, 950), or
the adhesive layer (230) is provided on the sensor unit housing (120) to be attached to the base unit (200, 960).
